# EUROPEAN PATENT APPLICATION

(11) **EP 4 567 113 A1**
(43) Date of publication of application: **11.06.2025**
(21) Application number: 23850083.9
(22) Date of filing: 01.08.2023
(51) Int. Cl.: C12N 15/12, C12N 5/14, C12N 15/82, C12P 21/02

(54) **COLLAGEN PRODUCTION METHOD**

(30) Priority: 02.08.2022 JP 2022123600
(71) Applicant: UniBio Corporation, Niigata-shi Niigata, 959-0597 (JP)
(72) Inventor: YOSHINAKA, Kyoji, Niigata-shi, Niigata 959-0597 (JP); NAKABAYASHI, Osamu, Niigata-shi, Niigata 959-0597 (JP); OKAWA, Shouichirou, Niigata-shi, Niigata 959-0597 (JP); YUKI, Masayuki, Niigata-shi, Niigata 959-0597 (JP)
(74) Representative: Nederlandsch Octrooibureau
(86) International application number: PCT/JP2023/028120
(87) International publication number: WO 2024/029529

(57) **Abstract**

The present disclosure relates to a method for producing a plant or an isolated plant cell that produces collagen, a nucleic acid construct for use in the method, and a method for producing a collagen protein using the plant or the isolated plant cell.

## Description

### Technical Field

The present disclosure relates to a method for producing a plant or an isolated plant cell that produces collagen, a nucleic acid construct for use in the method, and a method for producing a collagen protein using the plant or the isolated plant cell.

### Background Art

Collagen forms a family of extracellular matrix proteins present in all mammalian connective tissues, and a collagen molecule includes a structure in which three of polypeptides each called an α chain form a triple-helical region. Collagen such as type I collagen is involved in various biological reactions such as early development, organogenesis, cell adhesion, cell proliferation, hematopoiesis, wound healing and tissue remodeling in animals. Collagen, whose structural characteristics, low immunogenicity and the like have attracted attention, thus is widely used in the pharmaceutical field, the therapy field, the cosmetic field, the food fields and the like (Non Patent Literature 1: Ruggiero et al., 2000, FEBS Letter, 2000, Vol. 469, pp. 132-136 and Non Patent Literature 2: Merle et al., FEBS Letter, 2002, Vol. 515, pp. 114-118).

However, collagen that is used in these applications is generally extracted from animal tissues, and may be contaminated with viruses and other infectious substances. Therefore, in the industry in each of the above-described fields, the provision of a method for obtaining collagen from sources other than animal tissues is expected. As a cost-effective mass production method for mass-producing an animal protein with safety, an expression system with a genetically-modified plant has been used, and attempts have been made to produce collagen in plant bodies of genetically-modified nicotiana and the like (Non Patent Literature 2: Merle et al., FEBS Letter, 2002, Vol. 515, pp. 114-118 and Patent Literature 1: WO 2006/035442 A).

However, collagen is a protein having a complicated structure with a triple-helical structure formed by three peptide chains, and further technical improvement has been required for producing, in plant bodies, collagen that can be used in the pharmaceutical field, the therapy field, the cosmetic field, the food field and the like. For example, cells of plants for use in protein production, such as nicotiana plants, have an enzyme that cleaves an area which is off the cleavage site of a pro region intrinsic to a human, and high-quality collagen completely matching human collagen in structure has been difficult to produce by simply expressing collagen inside the plant cell. Therefore, collagen having quality acceptable in the pharmaceutical field, the therapy field, the cosmetic field, the food field and the like has been difficult to produce by simply expressing collagen inside the plant cell.

### Citation List

### Patent Literature

Patent Literature 1: WO 2006/035442

### Non Patent Literature

Non Patent Literature 1 Ruggiero et al., 2000, FEBS Letter, 2000, Vol. 469, pp. 132-136.
Non Patent Literature 2 Merle et al., FEBS Letter, 2002, Vol. 515, pp. 114-118.

### Summary of Invention

### Technical Problem

An object of the present disclosure is to provide a further improved method for producing collagen usable in the pharmaceutical field, the therapy field, the cosmetic field, the food field and the like in a plant or an isolated plant cell.

### Solution to Problem

The inventors of the present disclosure have extensively conducted tests and studies on a method for producing the collagen in a plant or an isolated plant cell, and resultantly succeeded in efficiently producing collagen in a plant or an isolated plant cell by finding that transient expression of procollagen inside the plant cell suppresses cleavage of the N-terminus of the procollagen in the plant body, leading to completion of the invention of the present application.

In one aspect, the present disclosure provides a method for producing procollagen or collagen from a plant body or an isolated plant cell, including the step of transiently expressing procollagen in the plant body or the isolated plant cell.

In one aspect, the present disclosure provides collagen or procollagen produced in a plant body or an isolated plant cell.

In one aspect, the present disclosure provides a nucleic acid construct for producing collagen or procollagen in a plant body or an isolated plant cell.

In one aspect, the present disclosure provides a vector containing a nucleic acid construct for producing collagen or procollagen in a plant body or an isolated plant cell, and a plant body or an isolated plant cell containing the vector.

In one aspect, the present disclosure provides procollagen or collagen which has a non-natural sequence and is encoded by
a nucleic acid construct for producing collagen or procollagen in a plant body or an isolated plant cell.

In one aspect, the present disclosure provides a composition containing procollagen or collagen.

More specifically, the present disclosure provides the following.

### [Item 1]

A method for producing procollagen or collagen from a plant body or an isolated plant cell, including the steps of:
A) providing a plant body or an isolated plant cell containing a nucleic acid construct encoding procollagen;
B) transiently expressing procollagen in the plant body or the isolated plant cell; and
C) separating procollagen or collagen secreted to the outside of the cell by step B.

### [Item 2]

A method for producing procollagen or collagen from a plant body or an isolated plant cell, including the step of cleaving an N-terminus or a C-terminus of procollagen secreted to the outside of the cell after step B.

### [Item 3]

The method according to item 1 or 2, wherein the nucleic acid construct contains a nucleic acid sequence encoding procollagen having an amino acid sequence identity of 80% or more to natural procollagen.

### [Item 4]

The method according to item 1 or 2, wherein the nucleic acid construct contains a nucleic acid sequence encoding the full length of procollagen and has a mutation which prevents the N-terminus of the procollagen from being cleaved inside the plant cell.

### [Item 5]

Procollagen or collagen produced by the method according to any one of items 1 to 4.

### [Item 6]

A nucleic acid construct which expresses collagen, and is used in the method according to any one of items 1 to 4.

### [Item 7]

An expression vector including the nucleic acid construct according to item 6.

### [Item 8]

A plant body or an isolated plant cell including the nucleic acid construct according to item 5 or the expression vector according to item 7.

### [Item 9]

Procollagen or collagen encoded by the nucleic acid construct according to item 6, the procollagen or collagen having a non-natural amino acid sequence.

### [Item 10]

The procollagen or collagen according to item 9, wherein a heterogeneous peptide is fused to an N-terminus pro region.

### [Item 11]

A composition that is a pharmaceutical composition, a cosmetic composition or a food composition, the composition including procollagen or collagen produced by the method according to any one of items 1 to 4, or the procollagen or collagen according to item 9 or 10.

### Advantageous Effects of Invention

The present disclosure has the effect of providing a further improved method for producing collagen usable in the pharmaceutical field, the therapy field, the cosmetic field, the food field and the like in a plant or an isolated plant cell.

### Brief Description of Drawings

Fig. 1a is a schematic diagram showing a configuration of a collagen type I α1 expression cassette.
Fig. 1b is a schematic diagram showing a configuration of a collagen type I α2 expression cassette.
Fig. 1c is a schematic diagram showing a configuration of a prolyl 4-hydroxylase α subunit (P4Hα)/prolyl 4-hydroxylase β subunit (P4Hβ) expression cassette.
Fig. 1d is a schematic diagram showing a configuration of a lysyl hydroxylase 3 (LH3) expression cassette.
Fig. 1e is a schematic diagram showing a configuration of a bone morphogenetic protein 1 (BMP1) expression cassette.
Fig. 1f is a schematic diagram showing a configuration of a Tomato bushy stunt virus-derived RNA silencing suppressor p19 expression cassette.

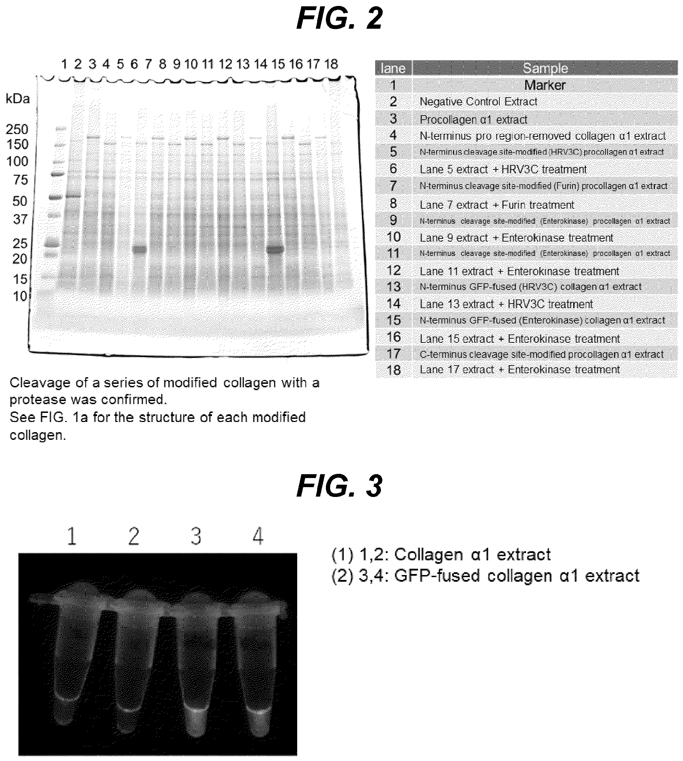

Fig. 2 shows the fact that a full-length collagen protein having an N-terminus pro region and a C-terminus pro region was confirmed to be accumulated in a plant body by transiently expressing a type I collagen α1 expression cassette in a Nicotiana benthamiana plant expression system. Lane 3 shows the fact that a full-length collagen protein expressed from the collagen type I α1 expression cassette was extracted. Lanes 4 to 18 shows the fact that full-length collagen proteins expressed from type I collagen α1 expression cassettes having cleavage site mutations corresponding to respective proteases were cleaved by the corresponding proteases.
Fig. 3 shows the fact that fluorescence was confirmed by irradiating an extract of a green fluorescent protein (GFP)-fused collagen α1 extract with excitation light.
Fig. 4 shows the results of a Western-blot experiment for confirming that α1 and α2 collagen proteins are simultaneously accumulated inside a cell body by co-expression of a type I collagen α1 expression cassette and a type I collagen α2 expression cassette in a plant expression system.
Fig. 5 shows the results of a test in which expression of α1 and α2 collagen proteins was confirmed by a mass spectrometer.

### Description of Embodiments

Collagen is composed of three peptide chains each called an α chains, and the peptides synthesized from mRNA by ribosomes bound to the endoplasmic reticulum membrane of cytoplasm move into the endoplasmic reticulum along with the synthesis, and undergo a S-S binding reaction at their respective C-termini to form a triple chain procollagen molecule consisting of three peptides. The triple chain procollagen molecule secreted from the endoplasmic reticulum to the outside of the cell through the Golgi body is cleaved at the N-terminus and the C-terminus to form mature triple chain collagen (Ricard-Blum, Cold Spring Harb Perspect Biol. 2011 Jan 1; 3(1): a004978).

The mature triple chain collagen is classified in various ways into type I collagen, type II collagen, type III to type XXVIII collagen, and the like depending on types and a combination of constituent α chains, and a structure or characteristics as a whole. For example, type I collagen is mainly composed of two α1 peptides and one α2 peptide, and type II collagen is composed of three α1 peptides. Each α chain is further varied by a difference in splicing mode during biosynthesis. Type III collagen is composed of three α1 peptides like type II collagen, but the splicing modes of the α chains forming the respective collagens are different (Ricard-Blum, Cold Spring Harb Perspect Biol. 2011 Jan 1; 3 (1): a004978).

Some types of mature triple chain collagens are further cross-linked to form a fiber structure, and some types of mature triple chain collagens form a network structure. These collagens are produced in various places in the living body, and have various biological functions (Ricard-Blum, Cold Spring Harb Perspect Biol. 2011 Jan 1; 3 (1): a004978).

For the collagen to be mass-produced for use in the pharmaceutical field, the therapy field, the cosmetic field, the food field and the like, the inventors of the present disclosure have extensively conducted tests and studies on a method for producing collagen in a plant or an isolated plant cell, and resultantly discovered that when the expression of procollagen inside the plant cell is transient, degradation of expressed collagen by an endogenous enzyme in the plant cell can be considerably suppressed. Further, the inventors have found that by introducing a mutation into a collagen gene, triple chain procollagen molecules or mature triple chain collagen molecules can be collected from a plant body, so that collagen can be efficiently produced.

More specifically, in a mammalian living body, natural collagen is secreted to the outside of the cell as procollagen having an extension region at the N-terminus and the C-terminus, and the N-terminus and the C-terminus are then cleaved by an enzyme to form a mature collagen molecule. On the other hand, when procollagen is expressed inside the plant cell, the N-terminus and the C-terminus are cleaved inside the plant cell, resulting in a considerable decrease in water solubility. The N-terminus cleavage site cleaved inside the plant cell is intrinsically located closer to the C-terminus than a site where the N-terminus is cleaved in maturation of procollagen into collagen, and mature collagen obtained from procollagen expressed inside the plant cell has an N-terminus shorter than that of natural mature collagen. In addition, the cleavage location of the N-terminus cleavage site that is cleaved inside the plant cell is unknown. Due to the cleavage at a non-natural location inside the plant cell, collagen produced inside the plant cell can have characteristics different from those of natural collagen.

The inventors of the present disclosure have discovered that when the expression of collagen inside the plant cell is transient, cleavage of procollagen at the N-terminus inside the plant cell can be suppressed. Further, the inventors have found amino acid mutations capable of suppressing cleavage of procollagen at the N-terminus or the C-terminus inside the plant cell or in a production process, and expressed procollagen having these mutations, thereby enabling efficient collection of procollagen or collagen secreted to the outside of the cell. Further, the inventors have cleaved the N-terminus and the C-terminus extension regions of procollagen, and introduced an enzyme recognition sequence into a collagen gene so that the amino acid sequences of the N-terminus and the C-terminus of the mature collagen molecule after the cleavage are identical to the amino acid sequence of a natural mature collagen molecule, thereby enabling conversion of the procollagen into a mature collagen molecule in a desired process.

### (Definitions)

Unless otherwise indicated, all technical and scientific terms used herein have the same meanings as commonly understood by those skilled in the art to which the present disclosure belongs.

Herein, when a plurality of ranges of numerical values are indicated, a range consisting of a combination of any lower limit and any upper limit in each of the plurality of ranges has the same meaning.

Herein, the term "substantially" has the same meaning as commonly understood by those skilled in the art to which the present disclosure belongs, but the term is used with the intention to include, for example, intended conditions and conditions inevitably unachievable due to biological or chemical characteristics, with consideration given to a possible case where a biological or chemical phenomenon does not completely achieve an intended condition.

Herein, when used in connection with a numerical value, the term "about" means that the value can vary within the range of, for example, 10%, 9%, 8%, 7%, 6%, 5%, 4%, 3%, 2%, 1%, 0.5%, 0.1%, 0.05% or 0.01%.

Herein, the term "include(s), comprise(s) or contain(s)" has the same meaning as commonly understood by those skilled in the art to which the present disclosure belongs, but includes, for example, "including, comprising or containing" and "consisting of". Specifically, a composition "including, comprising or containing" A may contain only A, or also contain another component B.

Herein, the term "consisting of" or "composed of" used in connection with a composition has the same meaning as commonly understood by those skilled in the art to which the present disclosure belongs, but the term is used to indicate a component that solely forms the composition. For example, a composition "consisting of" A contains only A. However, in one aspect, the term "composition "consisting of" A" includes a form that contains ingredients other than A which are unavoidable in production in terms of biological and chemical properties.

In the present disclosure, the term "identity" of amino acid sequences or nucleotide sequences means that two amino acid sequences or nucleotide sequences to be compared are aligned such that the number of matched amino acids or bases is as large as possible, the number of matched amino acids or bases is divided by the total number of amino acids or bases, and the quotient is expressed as a percentage. In the alignment, a gap is appropriately inserted into one or both of the two sequences to be compared as necessary. The sequences can be aligned using a well-known program such as BLAST, FASTA or CLUSTAL W. When a gap is inserted, the total number of amino acids or bases is a number counted with one gap considered as one amino acid or base. If the total number of amino acids or bases which is counted in this manner is different between two sequences to be compared, the identity (%) is calculated by dividing the number of matched amino acids or bases by the total number of amino acids or bases of the longer sequence. However, if a sequence to be compared is connected to another arbitrary sequence, only a corresponding region is taken and used to compare sequences, followed by calculation of the identity.

In the present disclosure, the phrase "expression of procollagen or collagen is "transient"" has the same meaning as commonly understood by those skilled in the art to which the present disclosure belongs, but the phrase means that procollagen or collagen is expressed inside the host cell typically in a state where a nucleic acid sequence encoding procollagen or collagen is not incorporated into the genome of the host cell. The transient expression can be performed by, for example, using a virus vector.

### (Method for producing collagen in plant or isolated plant cell)

In one aspect, the present disclosure provides a method for producing collagen in a plant or an isolated plant cell.

The collagen production method of the present disclosure includes the step of collecting procollagen or collagen molecules secreted to the outside of the cell by transiently expressing a nucleic acid construct containing a nucleic acid encoding procollagen in a plant body or an isolated plant cell, which finally leads to production of procollagen or collagen molecules.

Typically, the procollagen or collagen production method of the present disclosure includes the following steps of:
A) providing a plant body or an isolated plant cell containing a nucleic acid construct encoding procollagen;
B) transiently expressing procollagen in the plant body or the isolated plant cell; and
C) separating the procollagen or collagen secreted to outside of the cell by step B.

The nucleic acid construct for use in the step A) "providing a plant body or an isolated plant cell containing a nucleic acid construct encoding procollagen" is typically a nucleic acid construct having a configuration described in (Nucleic acid construct for use in production of collagen in plant or isolated plant cell) below.

The type of collagen produced in the procollagen or collagen production method of the present disclosure is not particularly limited, and arbitrary collagen such as type I collagen, type II collagen or type III collagen can be produced by combining appropriate nucleic acid constructs encoding an α chain. It is possible to produce, for example, collagen having a non-natural amino acid sequence or collagen to which a heterogeneous peptide is fused as described in (Procollagen or Collagen encoded by nucleic acid construct) below.

Examples of the plant for use in the procollagen or collagen production method of the present disclosure include Nicotiana plants, bryophyte plants (Physcomitrella patens (P. patens) and the like), potato (S. tuberosum and the like), gramineous plants (O. sativa and the like), cruciferous plants, and lettuce, with Nicotiana plants being preferable. Examples of the Nicotiana plant include, but are not limited to, Nicotiana benthamiana (N. benthamiana), Nicotiana tabacum (N. tabacum), and Nicotiana excelsior (N. excelsior).

In the step B) of "transiently expressing procollagen in a plant body or an isolated plant cell", the procollagen is expressed in a plant body or an isolated plant cell typically in a state where an expression vector containing a nucleic acid sequence encoding procollagen, such as a virus vector, is not incorporated into the genome of a host cell.

By transiently expressing procollagen, N-terminus cleavage inside the plant cell can be suppressed. Procollagen can be transiently expressed inside a plant body or an isolated plant cell by a method known to those skilled in the art to which the present disclosure belongs. Those skilled in the art can appropriately adjust the expression level of procollagen, the period of the expression and the like without departure from the spirit of the present disclosure by appropriately adjusting a vector to be used, a promoter, a configuration of a medium, culture conditions and the like.

For example, when the nucleic acid construct of the present disclosure is used as a recombinant virus, the virus grows inside a plant body or an isolated plant cell infected with the virus, and a large amount of collagen is produced inside the plant cell. For example, when collagen is produced using a plant body, collagen or procollagen can be extracted and collected from a plant tissue of a leaf or the like after cultivation of the plant for about 3 to 15 days after virus inoculation.

In the step C) of "separating procollagen or collagen secreted to the outside of the cell by step B", it is possible to use an arbitrary method known to those skilled in the art to which the present disclosure belongs. Examples that can be used include, but are not limited to, those described in C. D. Mount et al., Archives of Biochemistry and Biophysics, 240 33 (1985), U. H. Gregory and I. R. Willshire, Hoppe-Sayler's Z. Physiol. Chem., 356 1765 (1975). More specifically, for example, when collagen is expressed to produce collagen or procollagen in a nicotiana plant body, a leaf of a cryopreserved the nicotiana plant can be ground in an appropriate buffer, and collagen or procollagen can be extracted and purified from the buffer by salting-out.

In one aspect, the procollagen or collagen production method of the present disclosure may include the step of cleaving the N-terminus or C-terminus of procollagen secreted to the outside of the cell after step B. The cleavage can be performed by subjecting procollagen to chemical treatment, enzymatic treatment, or a combination thereof. At least a part of the chemical or enzymatic treatment is performed at a location other than the inside of a plant cell, and can be performed, for example, during the culture step, during the extraction and purification step, or after the extraction and purification step. For example, it is known that the C-terminus of natural procollagen can be removed by extracting and purifying full-length procollagen in an acidic environment. In one aspect, the N-terminus or the C-terminus of procollagen can be removed with a protease (proteolytic enzyme) after the extraction and purification step. By removing the N-terminus or the C-terminus of procollagen with a protease after the extraction and purification step to suppress ingress of contaminants into a removal reaction system, and carrying out a removal reaction under reaction conditions suitable for the protease, the specificity of the removal reaction can be improved to obtain high product stability. In this way, stable collagen can be produced and made into a final form of selling. In addition, there is a possible applied method in which with full-length procollagen as a raw material of a bio ink for a bio 3D printer, a three-dimensional structure is constructed with the bio 3D printer, and an enzyme that cleaves the N-terminus or the C-terminus is cleaved (for example, BMP1) is then added to further strengthen a triple-helical structure. This is a process corresponding to "development" of a photographic film before the spread of digital cameras.

The enzyme that cleaves the N-terminus or C-terminus extension region of the procollagen is not particularly limited. In one aspect, natural mature collagen can be produced from natural procollagen by using an enzyme that cleaves the N-terminus and the C-terminus of natural procollagen in mammalian cells. As a non-limiting example of the enzyme that cleaves the N-terminus and the C-terminus of natural procollagen, Procollagen N-Endopeptidase, BMP1 or the like can be used. In addition, when a desired protease recognition sequence is introduced to the N-terminus and C-terminus of procollagen using amino acid mutation, a corresponding protease can be used. Examples of the enzyme that can be used include, but are not limited to, HRV3C, Furin and Enterokinase. The recognition sequences and cleavage sites of HRV3C, Furin and Enterokinase are as follows.

| Protease | Recognition sequence and cleavage site (represented by "/") |
|---|---|
| HRV3C | Leu Glu Val Leu Phe Gln / Gly Pro |
| Furin | Arg Xaa Arg Arg / |
| | Arg Xaa Lys Arg / |
| Enterokinase | Asp Asp Asp Asp Lys / |

In one aspect, the procollagen or collagen production method of the present disclosure may include the step of post-translationally modifying procollagen or collagen. By post-translationally modifying procollagen or collagen with, for example, P4Hα, P4Hβ or LH3, the stability of procollagen or collagen can be improved. In the procollagen or collagen production method of the present disclosure, the step of performing post-translational modification can be carried out at any time point after translation of the procollagen or collagen, and can be carried out after secretion inside the plant cell or from the plant cell. For example, by expressing P4Hα and P4Hβ and procollagen in the same plant cell, post-translational modification can be performed inside the plant cell, for example, inside the endoplasmic reticulum. Preferably, at least a part of the post-translational modification is performed at a location other than the vacuole of the plant cell.

### (Collagen produced in plant or isolated plant cell)

In one aspect, the present disclosure provides procollagen or collagen produced in a plant or an isolated plant cell. Most of collagen generally provided heretofore in the pharmaceutical field, the cosmetic field, the food field or the like is obtained by decomposing, for example, collagen fibers in which collagen molecules present in living tissues are further polymerized. Such collagen loses a part of the structure and functions of collagen through decomposition treatment. On the other hand, procollagen or collagen produced in a plant or an isolated plant cell according to the present disclosure can be produced without undergoing the decomposition treatment, and thus can retain the structure and functions of collagen in a more complete state.

In one aspect, the procollagen produced by the production method of the present disclosure includes procollagen having an N-terminus and a C-terminus. The procollagen can be produced by transiently expressing a nucleic acid construct containing a nucleic acid encoding a natural procollagen inside the plant cell, and extracting and purifying procollagen secreted to the outside of the cell.

In one aspect, the procollagen produced in the present disclosure includes procollagen having a natural amino acid sequence. The procollagen having a natural sequence is identical in amino acid sequence to natural procollagen, but can differ in terms of a post-translational modification state or the like depending on various conditions in its production process. As described above, the method for producing collagen in a plant or an isolated plant cell according to the present disclosure is capable of controlling the post-translational modification state in various ways. This can provide various benefits related to physiological activity.

In one aspect, the procollagen produced in the present disclosure includes non-natural procollagen having an amino acid mutation at the N-terminus cleavage site or the C-terminus cleavage site or both. In one aspect, the mutation suppresses cleavage of procollagen inside the plant cell or during the production process. In one aspect, the mutation provides a recognition sequence corresponding to a desired protease. In one aspect, the mutation provides a sequence that induces cleavage through chemical treatment in the production process. The amino acid mutation at the N-terminus cleavage site or the C-terminus cleavage site of the non-natural full-length procollagen may be intended to finally produce native mature collagen, or may be intended to produce mature collagen having a non-natural sequence at the N-terminus or the C-terminus or both.

The amino acid mutation can be an addition, a deletion or a substitution of an amino acid, or a combination thereof with respect to a natural amino acid sequence.

In one aspect, the procollagen produced by the production method of the present disclosure includes N-terminus-absent procollagen and N-terminus-absent procollagen which are obtained by removing only the N-terminus or the C-terminus. The N-terminus-absent procollagen has an N-terminus-absent form that is different from that obtained by removal inside the plant cell. In one aspect, the N-terminus or C-terminus-absent procollagen can be produced by performing chemical treatment or enzymatic treatment or a combination thereof on full-length or non-full-length procollagen secreted to the outside of the cell.

In one aspect, the procollagen produced in the present disclosure includes procollagen in which a peptide having a specific function is fused to the N-terminus or the C-terminus or both. The peptide fused is not particularly limited, and may be a peptide derived from a heterogeneous protein or a newly synthesized non-natural peptide. It is possible to use, for example, a peptide that promotes secretion to the outside of the cell, an affinity peptide that can be used in purification of procollagen, a labeled peptide that generates fluorescence, and the like. In one aspect, the fused peptide can be fused can be fused to procollagen using a linker sequence known to those skilled in the art to which the present disclosure belongs. In one aspect, a part or the whole of the region of the fused peptide can be removed from the procollagen by a chemical or enzymatic reaction.

In one aspect, the mature collagen produced in the present disclosure has a natural amino acid sequence at the N-terminus and the C-terminus. In another aspect, the collagen produced in the present disclosure has a non-natural amino acid sequence at at least one of the N-terminus and/or the C-terminus. The non-natural amino acid sequence at the N-terminus is different from that produced by removal inside the plant cell.

In one aspect, the procollagen or collagen produced in the present disclosure includes procollagen or collagen having an arbitrary amino acid mutation, other than those described above. In one aspect, the procollagen or collagen produced in the present disclosure includes procollagen or collagen in which the whole or a part of the region of procollagen or collagen and other peptides are fused, other than those described above.

### (Nucleic acid constructs for use in collagen production in plant or isolated plant cell)

In one aspect, the present disclosure provides a nucleic acid construct for use in collagen production in a plant or an isolated plant cell.

The nucleic acid construct of the present disclosure has a nucleic acid sequence encoding at least one of α chains forming collagen. In one aspect, the nucleic acid construct of the present disclosure has a sequence that expresses procollagen. The procollagen includes procollagen having various mutations and procollagen fused with other peptides, which are described in "Collagen produced in plant or isolated plant cell" above. The type of α chain encoded by the nucleic acid construct of the present disclosure is not particularly limited, and can encode any of α1, α2, and splice isomers thereof.

The nucleic acid construct of the present disclosure may be DNA, RNA or an arbitrary analog thereof known to those skilled in the art to which this disclosure belongs. For example, the nucleic acid construct of the present disclosure may include a non-natural base, and may include a hydrocarbon chain and a peptide chain in a part thereof.

The nucleic acid construct of the present disclosure can be prepared by a conventional chemical synthesis method known to those skilled in the art to which the present disclosure belongs, or a genetic engineering technique. It is also possible to synthesize the nucleic acid construct by, for example, synthesizing cDNA having a naturally occurring sequence, and then appropriately introducing a mutation using an appropriate primer with the cDNA as a template. Alternatively, a DNA construct can be synthesized by dividing a nucleic acid construct to be synthesized into some short regions, chemically synthesizing a plurality of DNA fragments, and connecting the fragments by a known fusion PCR method. A RNA construct can be prepared by a transcription reaction from the DNA construct.

As a method for introducing a mutation into the sequence of the nucleic acid construct, an arbitrary method known to those skilled in the art to which the present disclosure belongs can be used. Examples of the method that can be used by those skilled in the art include, but are not limited to, site-directed mutagenesis methods such as a Kunkel method or a Gapped duplex method, an overlap extension PCR method, and a QuikChange method.

In one aspect, the nucleic acid construct of the present disclosure may contain nucleic acid fragments for transient expression inside a plant body. For example, the nucleic acid construct may contain nucleic acid fragments encoding, for example, an appropriate RNA polymerase promoter (for example, a phage polymerase promoter such as a T7 promoter), a viral replicase, a movement protein or a coat protein which is required in synthesis of recombinant virus RNA by a transcription reaction.

In one aspect, the nucleic acid construct of the present disclosure may contain nucleic acid fragments encoding, for example, an enzyme useful for stably expressing collagen inside a plant body. For example, the nucleic acid construct may contain nucleic acid fragments encoding, for example, P4Hα, P4Hβ or LH3 that enhance the stability of collagen, or a combination thereof. As these enzymes, enzymes derived from various organisms can be used, and for example, enzymes derived from nematodes, mice, humans and the like can be used.

In one aspect, a part or all of the nucleic acid fragments encoding these enzymes and the like may be contained in a second nucleic acid construct different from a nucleic acid construct containing a nucleic acid encoding the α chains of procollagen, and the first nucleic acid construct containing a nucleic acid encoding the α chains of procollagen may be used in combination with the second nucleic acid construct.

When the first nucleic acid construct containing a nucleic acid encoding the α chains of procollagen and the second nucleic acid construct containing a nucleic acid fragment encoding an enzyme or the like are used in combination, the nucleic acid constructs may be introduced into the same plant body or isolated plant cell, or may be introduced into different plant bodies or isolated plant cells, so that the procollagen secreted to the outside of the cell and the enzyme react with each other.

### (Expression vector containing nucleic acid construct)

In one aspect, the present disclosure provides a vector containing a nucleic acid construct for use in collagen production in a plant or an isolated plant cell. The vector is used to transiently express collagen inside the plant host, and is typically provided as a plant virus vector. In one aspect, the expression vector of the present disclosure can be provided in the form of a "recombinant virus". The nucleic acid construct of the present disclosure is RNA when the virus is a RNA virus, and the nucleic acid construct is DNA when the virus is a DNA virus. In one aspect, it is also possible to transform the vector plasmid into Agrobacterium tumefaciens (Agrobacterium), which is transmitted to a plant to transiently express an intended protein.

The expression vector provided as a recombinant virus according to the present disclosure can be produced by conventionally modifying an arbitrary plant virus vector known to those skilled in the art to which the present disclosure belongs (see, for example, "Protein, Nucleic Acid, Enzyme", vol. 45, p. 607-613). In one aspect, it is preferable to modify the tomato bushy stunt virus into a recombinant virus.

### (Plant body or isolated plant cell containing nucleic acid construct or expression vector)

In one aspect, the present disclosure provides a plant body or an isolated plant cell containing a nucleic acid construct or an expression vector. The plant body or the isolated plant cell may be subjected to a treatment for introduction of a nucleic acid construct or an expression vector, or may be obtained by growth or proliferation of the treated plant body or plant cell.

### (Procollagen or collagen encoded by nucleic acid construct)

In one aspect, the present disclosure includes procollagen or collagen that is encoded by the nucleic acid construct of the present disclosure. The collagen or procollagen is not limited to collagen or procollagen produced in a plant body or an isolated plant cell. The procollagen or collagen typically has a non-natural amino acid sequence. In one aspect, the procollagen or collagen of the present disclosure has an amino acid sequence identity of 70% or more, 75% or more, 80% or more, 85% or more, 90% or more or 95% or more to natural procollagen or collagen. The procollagen or collagen of the present disclosure may include a substitution, an insertion or a deletion of one or more amino acids with respect to natural procollagen or collagen.

In one aspect, the procollagen or collagen of the present disclosure can be fused to a heterogenous protein instead of the N-terminus pro region, whereby various functions can be added. For example, fluorescent collagen can be produced by fusing GFP instead of the N-terminus pro region. The procollagen or collagen can be expressed and purified by arbitrary methods known to those skilled in the art to which the present disclosure belongs, and the procollagen or collagen can be used for the purpose of, for example, analyzing chemical characteristics and physiological activity.

### (Composition containing procollagen or collagen)

In one aspect, the present disclosure provides a composition containing procollagen or collagen produced from a plant body or an isolated plant cell, or procollagen or collagen having a non-natural amino acid sequence, which is produced by other methods.

In one aspect, the form of the compositions of the present disclosure is not limited, and is provided in the form of, for example, a pharmaceutical composition, a cosmetic composition or a food composition. The composition may contain, in addition to procollagen or collagen, a carrier, an excipient and the like that are acceptable for the composition in each of the fields. In the composition of the present disclosure, the procollagen or collagen may be intermolecularly cross-linked to form a multimer having a fibrous or network-like structure including a plurality of procollagen or collagen molecules. The amount of collagen contained in the composition of the present disclosure is not particularly limited, and is appropriately adjusted according to a dosage form, a purpose and the like.

The pharmaceutical composition containing procollagen or collagen according to the present disclosure includes a composition which itself is a pharmaceutical product, and a composition which is a raw material or an intermediate product in production of a pharmaceutical product. The form of the pharmaceutical composition of the present disclosure is not particularly limited, and may be a solid form, a powder form, a granular form, or a liquid form. When the pharmaceutical composition of the present disclosure is in a form to be administered to a subject, the dosage form thereof is not particularly limited, and may be provided as, for example, a tablet, a fine granule, a pill, a troche, a capsule, or a raw material or intermediate product thereof.

The pharmaceutical composition of the present disclosure includes a composition which is a medicament for regenerative medicine, or a composition which is a raw material or an intermediate product in production of the medicament. For example, the pharmaceutical composition of the present disclosure is provided as a sheet-like composition, and can be provided as a composition for promoting tissue regeneration inside the body or on body surface of a subject, or as a scaffold for culturing a sheet-like tissue. In one aspect, the pharmaceutical composition of the present disclosure can be provided as a raw material for a material composition for forming a regenerative medical product having a three-dimensional structure using a 3D printing technique or the like, for example, a bio ink.

In one aspect, the pharmaceutical composition of the present disclosure includes a composition that is an implantable medical device, or a raw material or intermediate product in production of the medical device. These compositions can be used for prevention or treatment of diseases or disorders, cosmetic purposes, and the like. Non-limiting examples of the composition include wound dressing materials, artificial skin, artificial blood vessels, catheters and other devices for removal of fluid or delivery of fluid to a patient, artificial hearts, artificial kidneys, and orthopedic pins, plates and implants. For example, the pharmaceutical compositions of the present disclosure can be used as 3D bio-printed post-mastectomy implants, and fillers for cosmetic surgery.

The cosmetic composition containing procollagen or collagen according to the present disclosure includes a composition which itself is a cosmetic, and a composition which is a raw material or an intermediate product in production of a cosmetic. The form of the cosmetic composition of the present disclosure is not particularly limited, and may be a solid form, a powder form, a granular form, or a liquid form. The cosmetic of the present disclosure may be topically applied or orally administered, and can be provided as, for example, a topically administered agent such as an ointment, a lotion, a cream, a microemulsion, a gel, an oil or a solution, an orally administered agent such as a tablet, a fine granule, a pill, a troche or a capsule, or a raw material or intermediate product thereof.

The food composition containing procollagen or collagen according to the present disclosure includes a composition which itself is a food product, and a composition which is a raw material or an intermediate product in production of a food product. The food includes health food, functional food, food for specified health use, and food for the sick, and feed when the food is intended for animals other than humans. The form of the food is not particularly limited, and may be a solid form, or a liquid form. The animal other than humans is not particularly limited, and various animals such as mammals, birds, reptiles, amphibians, fish, and insects can be covered. Specific examples of the type of food include, but are not limited to, beverages such as soft drinks, carbonated beverages, nutritional beverages, fruit beverages, and milk beverages, or liquid concentrates and adjustment powder for these beverages; frozen desserts such as ice cream, ice sherbet and shaved ice; noodles such as buckwheat noodles, wheat noodles, bean-starch vermicelli, gyoza skins, shumai skins, Chinese noodles and instant noodles; confectioneries such as drops, chewing gum, candy, gummy, gum, caramel, chocolate, tablet confectionery, snack confectionery, baked confectionery such as biscuits, jelly, jam, and cream; fish/livestock processed foods such as kamaboko, hamburger, ham and sausage; dairy products such as processed milk, fermented milk, yogurt, butter and cheese; oil/fat and oil/fat processed foods such as salad oil, temperate oil, margarine, mayonnaise, shortening, whipped cream and dressing; seasonings such as sauce and baste; soup, stew, curry, bread, jam, salad, daily dish and Japanese pickles.

Hereinafter, the present disclosure will be described in more detail by way of Examples, but these Examples are illustrative and do not limit the scope of the present disclosure to the scope of examples.

### Examples

### (Materials and methods)

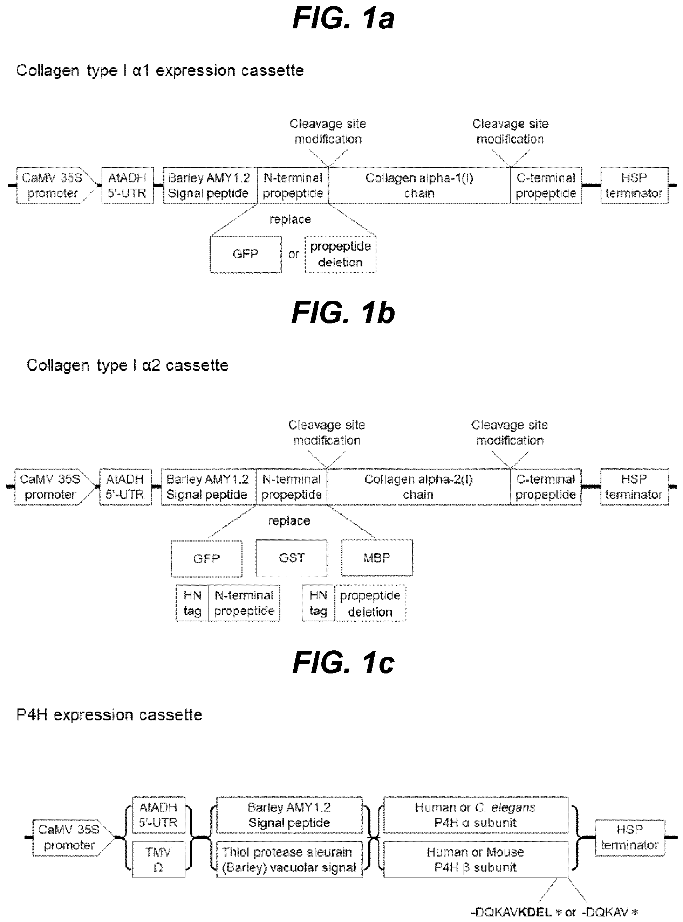

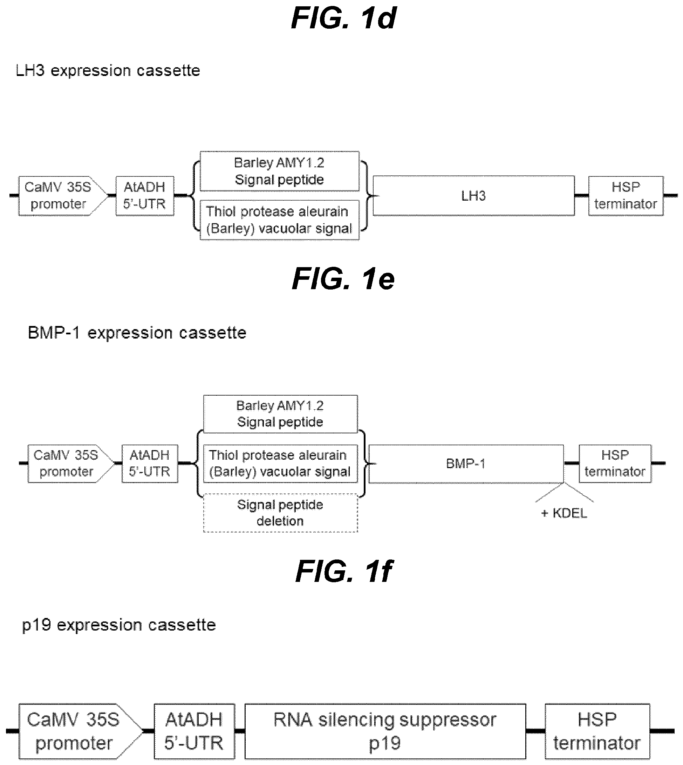

The construction of expression cassettes used in the present study is shown in Figs. 1a to 1f. The coding sequences in the present study were optimized for expression in nicotiana as necessary, and chemically synthesized to have a desirable flanking region.

The sequences used are shown below.

**[Table 1]**

| SEQ ID NO | Sequence name | Sequence |
|---|---|---|
| 1 | CaMV 35S Promoter (DNA sequence) | TGAGACTTTTCAACAAAGGGTAATATCCGGAAAC CTCCTCGGATTCCATTGCCCAGCTATCTGTCACT TTATTGTGAAGATAGTGGAAAAGGAAGGTGGCTC CTACAAATGCCATCATTGCGATAAAGGAAAGGCC ATCGTTGAAGATGCCTCTGCCGACAGTGGTCCCA AAGATGGACCCCCACCCACGAGGAGCATCGTGGA AAAAGAAGACGTTCCAACCACGTCTTCAAAGCAAG TGGATTGATGTGATATCTCCACTGACGTAAGGGA TGACGCACAATCCCACTATCCTTCGCAAGACCCTT CCTCTATATAAGGAAGTTCATTTCATTTGGAGAG AACA |
| 2 | TMV omega (DNA sequence) | ACAATTACCAACAACAACAAACAACAAACAACATT ACAATTACTATTTACAATTAC |
| 3 | AtADH 5'-UTR (DNA sequence) | TACATCACAATCACACAAAACTAACAAAAGATCAA AAGCAAGTTCTTCACTGTTGATA |
| 4 | HSP-terminator (DNA sequence) | TATGAAGATGAAGATGAAATATTTGGTGTGTCAA ATAAAAAGCTAGCTTGTGTGCTTAAGTTTGTGTT TTTTTCTTGGCTTGTTGTGTTATGAATTTGTGGC TTTTTCTAATATTAAATGAATGTAAGATCTCATTA TAATGAATAAACAAATGTTTCTATAATCCATTGTG AATGTTTTGTTGGATCTCTTCGCATATAACTACT GTATGTGCTATGGTATGGACTATGGAATATGATT AAAGATAAG |
| 5 | Barley AMY 1.2 Signal | MANKHLSLSLFLVLLGLSASLASG |
| | Peptide (amino acid sequence) | |
| 6 | Thiol protease aleurain (Barley) vacuolar signal (amino acid sequence) | MAHARVLLLALAVLATAAVAVASSSSFADSNPIRPVT DRAASTL |
| 7 | Collagen alpha-1 N-terminal propeptide (amino acid sequence) | QEEGQVEGQDEDIPPITCVQNGLRYHDRDVWKPEP CRICVCDNGKVLCDDVICDETKNCPGAEVPEGECCP VCPDGSESPTDQETTGVEGPKGDTGPRGPRGPAGP PGRDGIPGQPGLPGPPGPPGPPGPPGLGGNFAP |
| 8 | Collagen alpha-1 (I) chain (amino acid sequence) | QEEGQVEGQDEDIPPITCVQNGLRYHDRDVWKPEP CRICVCDNGKVLCDDVICDETKNCPGAEVPEGECCP VCPDGSESPTDQETTGVEGPKGDTGPRGPRGPAGP PGRDGIPGQPGLPGPPGPPGPPGPPGLGGNFAPQL SYGYDEKSTGGISVPGPMGPSGPRGLPGPPGAPGPQ GFQGPPGEPGEPGASGPMGPRGPPGPPGKNGDDG EAGKPGRPGERGPPGPQGARGLPGTAGLPGMKGH RGFSGLDGAKGDAGPAGPKGEPGSPGENGAPGQM GPRGLPGERGRPGAPGPAGARGNDGATGAAGPPGP TGPAGPPGFPGAVGAKGEAGPQGPRGSEGPQGVRG EPGPPGPAGAAGPAGNPGADGQPGAKGANGAPGIA GAPGFPGARGPSGPQGPGGPPGPKGNSGEPGAPGS KGDTGAKGEPGPVGVQGPPGPAGEEGKRGARGEP GPTGLPGPPGERGGPGSRGFPGADGVAGPKGPAGE RGSPGPAGPKGSPGEAGRPGEAGLPGAKGLTGSPGS |
| | | PGPDGKTGPPGPAGQDGRPGPPGPPGARGQAGVM GFPGPKGAAGEPGKAGERGVPGPPGAVGPAGKDGE AGAQGPPGPAGPAGERGEQGPAGSPGFQGLPGPAG PPGEAGKPGEQGVPGDLGAPGPSGARGERGFPGER GVQGPPGPAGPRGANGAPGNDGAKGDAGAPGAPG SQGAPGLQGMPGERGAAGLPGPKGDRGDAGPKGA DGSPGKDGVRGLTGPIGPPGPAGAPGDKGESGPSG PAGPTGARGAPGDRGEPGPPGPAGFAGPPGADGQP GAKGEPGDAGAKGDAGPPGPAGPAGPPGPIGNVGA PGAKGARGSAGPPGATGFPGAAGRVGPPGPSGNAG PPGPPGPAGKEGGKGPRGETGPAGRPGEVGPPGPP GPAGEKGSPGADGPAGAPGTPGPQGIAGQRGVVGL PGQRGERGFPGLPGPSGEPGKQGPSGASGERGPPG PMGPPGLAGPPGESGREGAPGAEGSPGRDGSPGAK GDRGETGPAGPPGAPGAPGAPGPVGPAGKSGDRGE TGPAGPTGPVGPVGARGPAGPQGPRGDKGETGEQ GDRGIKGHRGFSGLQGPPGPPGSPGEQGPSGASGP AGPRGPPGSAGAPGKDGLNGLPGPIGPPGPRGRTG DAGPVGPPGPPGPPGPPGPPSAGFDFSFLPQPPQEK AHDGGRYYRADDANVVRDRDLEVDTTLKSLSQQIE NIRSPEGSRKNPARTCRDLKMCHSDWKSGEYWIDPN QGCNLDAIKVFCNMETGETCVYPTQPSVAQKNWYI SKNPKDKRHVWFGESMTDGFQFEYGGQGSDPADV AIQLTFLRLMSTEASQNITYHCKNSVAYMDQQTGNL KKALLLQGSNEIEIRAEGNSRFTYSVTVDGCTSHTGA WGKTVIEYKTTKTSRLPIIDVAPLDVGAPDQEFGFD VGPVCFL |
| 9 | Collagen alpha-1 C-terminal propeptide (amino | DDANVVRDRDLEVDTTLKSLSQQIENIRSPEGSRKN PARTCRDLKMCHSDWKSGEYWIDPNQGCNLDAIKV FCNMETGETCVYPTQPSVAQKNWYISKNPKDKRHV WFGESMTDGFQFEYGGQGSDPADVAIQLTFLRLMS TEASQNITYHCKNSVAYMDQQTGNLKKALLLQGSN |
| | acid sequence) | EIEIRAEGNSRFTYSVTVDGCTSHTGAWGKTVIEYKT TKTSRLPIIDVAPLDVGAPDQEFGFDVGPVCFL |
| 10 | Collagen alpha-2 N-terminal propeptide (amino acid sequence) | QSLQEETVRKGPAGDRGPRGERGPPGPPGRDGEDG PTGPPGPPGPPGPPGLGGNFAA |
| 11 | Collagen alpha-2 (I) chain (amino acid sequence) | QSLQEETVRKGPAGDRGPRGERGPPGPPGRDGEDG PTGPPGPPGPPGPPGLGGNFAAQYDGKGVGLGPGP MGLMGPRGPPGAAGAPGPQGFQGPAGEPGEPGQT GPAGARGPAGPPGKAGEDGHPGKPGRPGERGVVG PQGARGFPGTPGLPGFKGIRGHNGLDGLKGQPGAP GVKGEPGAPGENGTPGQTGARGLPGERGRVGAPG PAGARGSDGSVGPVGPAGPIGSAGPPGFPGAPGPKG EIGAVGNAGPAGPAGPRGEVGLPGLSGPVGPPGNP GANGLTGAKGAAGLPGVAGAPGLPGPRGIPGPVGA AGATGARGLVGEPGPAGSKGESGNKGEPGSAGPQG PPGPSGEEGKRGPNGEAGSAGPPGPPGLRGSPGSRG LPGADGRAGVMGPPGSRGASGPAGVRGPNGDAGR PGEPGLMGPRGLPGSPGNIGPAGKEGPVGLPGIDGR PGPIGPAGARGEPGNIGFPGPKGPTGDPGKNGDKG HAGLAGARGAPGPDGNNGAQGPPGPQGVQGGKG EQGPPGPPGFQGLPGPSGPAGEVGKPGERGLHGEF GLPGPAGPRGERGPPGESGAAGPTGPIGSRGPSGPP GPDGNKGEPGVVGAVGTAGPSGPSGLPGERGAAGI PGGKGEKGEPGLRGEIGNPGRDGARGAPGAVGAPG PAGATGDRGEAGAAGPAGPAGPRGSPGERGEVGPA GPNGFAGPAGAAGQPGAKGERGAKGPKGENGVVG PTGPVGAAGPAGPNGPPGPAGSRGDGGPPGMTGF PGAAGRTGPPGPSGISGPPGPPGPAGKEGLRGPRGD QGPVGRTGEVGAVGPPGFAGEKGPSGEAGTAGPPG |
| | | TPGPQGLLGAPGILGLPGSRGERGLPGVAGAVGEPG PLGIAGPPGARGPPGAVGSPGVNGAPGEAGRDGNP GNDGPPGRDGQPGHKGERGYPGNIGPVGAAGAPG PHGPVGPAGKHGNRGETGPSGPVGPAGAVGPRGPS GPQGIRGDKGEPGEKGPRGLPGLKGHNGLQGLPGI AGHHGDQGAPGSVGPAGPRGPAGPSGPAGKDGRT GHPGTVGPAGIRGPQGHQGPAGPPGPPGPPGPPGV SGGGYDFGYDGDFYRADQPRSAPSLRPKDYEVDATL KSLNNQIETLLTPEGSRKNPARTCRDLRLSHPEWSSG YYWIDPNQGCTMDAIKVYCDFSTGETCIRAQPENIP AKNWYRSSKDKKHVWLGETINAGSQFEYNVEGVTS KEMATQLAFMRLLANYASQNITYHCKNSIAYMDEET GNLKKAVILQGSNDVELVAEGNSRFTYTVLVDGCSK KTNEWGKTIIEYKTNKPSRLPFLDIAPLDIGGADQEF FVDIGPVCFK |
| 12 | Collagen alpha-2 C-terminal propeptide (amino acid sequence) | DQPRSAPSLRPKDYEVDATLKSLNNQIETLLTPEGSR KNPARTCRDLRLSHPEWSSGYYWIDPNQGCTMDAI KVYCDFSTGETCIRAQPENIPAKNWYRSSKDKKHVW LGETINAGSQFEYNVEGVTSKEMATQLAFMRLLANY ASQNITYHCKNSIAYMDEETGNLKKAVILQGSNDVE LVAEGNSRFTYTVLVDGCSKKTNEWGKTIIEYKTNK PSRLPFLDIAPLDIGGADQEFFVDIGPVCFK |
| 13 | GFP (amino acid sequence) | SKGEELFTGVVPILVELDGDVNGHKFSVSGEGEGDA TYGKLTLKFICTTGKLPVPWPTLVTTFSYGVQCFSRY PDHMKQHDFFKSAMPEGYVQERTIFFKDDGNYKTR AEVKFEGDTLVNRIELKGIDFKEDGNILGHKLEYNYN SHNVYIMADKQKNGIKVNFKIRHNIEDGSVQLADHY QQNTPIGDGPVLLPDNHYLSTQSALSKDPNEKRDH MVLLEFVTAAGITHGMDELYK |
| 14 | GST (amino acid sequence) | SPILGYWKIKGLVQPTRLLLEYLEEKYEEHLYERDEG DKWRNKKFELGLEFPNLPYYIDGDVKLTQSMAIIRYI ADKHNMLGGCPKERAEISMLEGAVLDIRYGVSRIAYS |
| | | KDFETLKVDFLSKLPEMLKMFEDRLCHKTYLNGDH VTHPDFMLYDALDVVLYMDPMCLDAFPKLVCFKKR IEAIPQIDKYLKSSKYIAWPLQGWQATFGGGDHPPK |
| 15 | MBP (amino acid sequence) | KIEEGKLVIWINGDKGYNGLAEVGKKFEKDTGIKVT VEHPDKLEEKFPQVAATGDGPDIIFWAHDRFGGYA QSGLLAEITPDKAFQDKLYPFTWDAVRYNGKLIAYPI AVEALSLIYNKDLLPNPPKTWEEIPALDKELKAKGKS ALMFNLQEPYFTWPLIAADGGYAFKYENGKYDIKDV GVDNAGAKAGLTFLVDLIKNKHMNADTDYSIAEAAF NKGETAMTINGPWAWSNIDTSKVNYGVTVLPTFKG QPSKPFVGVLSAGINAASPNKELAKEFLENYLLTDEG LEAVNKDKPLGAVALKSYEEELVKDPRIAATMENAQ KGEIMPNIPQMSAFWYAVRTAVINAASGRQTVDEAL KDAQTNSSSNNNNNNNNNNNLG |
| 16 | HN Tag (amino acid sequence) | HNHNHNHNHNHN |
| 17 | Human P4H alpha subunit (amino acid sequence) | HPGFFTSIGQMTDLIHTEKDLVTSLKDYIKAEEDKLE QIKKWAEKLDRLTSTATKDPEGFVGHPVNAFKLMK RLNTEWSELENLVLKDMSDGFISNLTIQRQYFPNDE DQVGAAKALLRLQDTYNLDTDTISKGNLPGVKHKS FLTAEDCFELGKVAYTEADYYHTELWMEQALRQLD EGEISTIDKVSVLDYLSYAVYQQGDLDKALLLTKKLL ELDPEHQRANGNLKYFEYIMAKEKDVNKSASDDQS DQKTTPKKKGVAVDYLPERQKYEMLCRGEGIKMTP RRQKKLFCRYHDGNRNPKFILAPAKQEDEWDKPRII RFHDIISDAEIEIVKDLAKPRLRRATISNPITGDLETVH YRISKSAWLSGYENPVVSRINMRIQDLTGLDVSTAEE LQVANYGVGGQYEPHFDFARKDEPDAFKELGTGNR IATWLFYMSDVSAGGATVFPEVGASVWPKKGTAVF WYNLFASGEGDYSTRHAACPVLVGNKWVSNKWLHE RGQEFRRPCTLSELE |
| 18 | C. elegans P4H alpha subunit (amino acid sequence) | DLFTSIADMQNLLETERNIPKILDKYIHDEEERLVQL KKLSEEYSKKNEISIENGLKDITNPINAFLLIKRKIFDW KEIESKMNANKAGNVVSSITDDSYGVRYPTADDLSG AAIGLLRLQDTYRLDTKDLADGKIYADQGNYTFSAK DCFEIARAAYNEHDFYHTVMWMEEAQRRLGDEVEP TVEVEDILEYLAFALYKQNNLKHALKLTEELYKMNP THPRAKGNVKWYEDLLEQEGVRRSDMRKNLPPIQN RRPDSVLGNTERTMYEALCRNEVPVSQKDISRLYCYY KRDRPFLVYAPIKVEIKRFNPLAVLFKDVISDDEVAAI QELAKPKLARATVHDSVTGKLVTATYRISKSAWLKE WEGDVVETVNKRIGYMTNLEMETAEELQIANYGIG GHYDPHFDHAKKEESKSFESLGTGNRIATVLFYMSQ PSHGGGTVFTEAKSTILPTKNDALFWYNLYKQGDG NPDTRHAACPVLVGIKWVSNKWIHEKGNEFRRPCG LKSSDYERFVGDLGYGPEPRNAPNVSPNLAKDVWET L |
| 1.9 | Human P4H beta subunit (amino acid sequence) | DAPEEEDHVLVLRKSNFAEALAAHKYLLVEFYAPWC GHCKALAPEYAKAAGKLKAEGSEIRLAKVDATEESDL AQQYGVRGYPTIKFFRNGDTASPKEYTAGREADDIV NWLKKRTGPAATTLPDGAAAESLVESSEVAVIGFFK DVESDSAKQFLQAAEAIDDIPFGITSNSDVFSKYQLD KDGVVLFKKFDEGRNNFEGEVTKENLLDFIKHNQLP LVIEFTEQTAPKIFGGEIKTHILLFLPKSVSDYDGKLS NFKTAAESFKGKILFIFIDSDHTDNQRILEFFGLKKEE CPAVRLITLEEEMTKYKPESEELTAERITEFCHRFLEG KIKPHLMSQELPEDWDKQPVKVLVGKNFEDVAFDE KKNVFVEFYAPWCGHCKQLAPIWDKLGETYKDHEN IVIAKMDSTANEVEAVKVHSFPTLKFFPASADRTVID YNGERTLDGFKKFLESGGQDGAGDDDDLEDLEEAE EPDMEEDDDQKAV |
| 20 | Mouse P4H beta | DALEEEDNVLVLKKSNFEEALAAHKYLLVEFYAPWC GHCKALAPEYAKAAAKLKAEGSEIRLAKVDATEESDL |
| | subunit (amino acid sequence) | AQQYGVRGYPTIKFFKNGDTASPKEYTAGREADDIV NWLKKRTGPAATTLSDTAAAESLVDSSEVTVIGFFK DVESDSAKQFLLAAEAIDDIPFGITSNSGVFSKYQLDK DGVVLFKKFDEGRNNFEGEITKEKLLDFIKHNQLPL VIEFTEQTAPKIFGGEIKTHILLFLPKSVSDYDGKLSSF KRAAEGFKGKILFIFIDSDHTDNQRILEFFGLKKEECP AVRLITLEEEMTKYKPESDELTAEKITEFCHRFLEGKI KPHLMSQEVPEDWDKQPVKVLVGANFEEVAFDEKK NVFVEFYAPWCGHCKQLAPIWDKLGETYKDHENIII AKMDSTANEVEAVKVHSFPTLKFFPASADRTVIDYN GERTLDGFKKFLESGGQDGAGDDEDLDLEEALEPD MEEDDDQKAVKDEL |
| 21 | LH3 (amino acid sequence) | SDRPRGRDPVNPEKLLVITVATAETEGYLRFLRSAEF FNYTVRTLGLGEEWRGGDVARTVGGGQKVRWLKK EMEKYADREDMIIMFVDSYDVILAGSPTELLKKFVQS GSRLLFSAESFCWPEWGLAEQYPEVGTGKRFLNSGG FIGFATTIHQIVRQWKYKDDDDDQLFYTRLYLDPGL REKLSLNLDHKSRIFQNLNGALDEVVLKFDRNRVRIR NVAYDTLPIVVHGNGPTKLQLNYLGNYVPNGWTPE GGCGFCNQDRRTLPGGQPPPRVFLAVFVEQPTPFL PRFLQRLLLLDYPPDRVTLFLHNNEVFHEPHIADSW PQLQDHFSAVKLVGPEEALSPGEARDMAMDLCRQD PECEFYFSLDADAVLTNLQTLRILIEENRKVIAPMLSR HGKLWSNFWGALSPDEYYARSEDYVELVQRKRVGV WNVPYISQAYVIRGDTLRMELPQRDVFSGSDTDPD MAFCKSFRDKGIFLHLSNQHEFGRLLATSRYDTEHL HPDLWQIFDNPVDWKEQYIHENYSRALEGEGIVEQP CPDVYWFPLLSEQMCDELVAEMEHYGQWSGGRHE DSRLAGGYENVPTVDIHMKQVGYEDQWLQLLRTYV GPMTESLFPGYHTKARAVMNFVVRYRPDEQPSLRP HHDSSTFTLNVALNHKGLDYEGGGCRFLRYDCVISS PRKGWALLHPGRLTHYHEGLPTTWGTRYIMVSFVD |
| | | P |
| 22 | BMP1 (amino acid sequence) | AATSRPERVWPDGVIPFVIGGNFTGSQRAVFRQAMR HWEKHTCVTFLERTDEDSYIVFTYRPCGCCSYVGRR GGGPQAISIGKNCDKFGIVVHELGHVVGFWHEHTRP DRDRHVSIVRENIQPGQEYNFLKMEPQEVESLGETY DFDSIMHYARNTFSRGIFLDTIVPKYEVNGVKPPIGQ RTRLSKGDIAQARKLYKCPACGETLQDSTGNFSSPEY PNGYSAHMHCVWRISVTPGEKIILNFTSLDLYRSRLC WYDYVEVRDGFWRKAPLRGRFCGSKLPEPIVSTDSR LWVEFRSSSNWVGKGFFAVYEAICGGDVKKDYGHIQ SPNYPDDYRPSKVCIWRIQVSEGFHVGLTFQSFEIER HDSCAYDYLEVRDGHSESSTLIGRYCGYEKPDDIKST SSRLWLKFVSDGSINKAGFAVNFFKEVDECSRPNRGG CEQRCLNTLGSYKCSCDPGYELAPDKRRCEAACGGF LTKLNGSITSPGWPKEYPPNKNCIWQLVAPTQYRISL QFDFFETEGNDVCKYDFVEVRSGLTADSKLHGKFCG SEKPEVITSQYNNMRVEFKSDNTVSKKGFKAHFFSEK RPALQPPRGRPHQLKFRVQKRNRTPQ |
| 23 | RNA silencing suppressor p19 (amino acid sequence) | MERAIQGNDAREQANSERWDGGSGSSTSPFQLPDES PSWTEWRLHNDETNSNQDNPLGFKESWGFGKVVF KRYLRYERTETSLHRVLGSWTGDSVNYAASRFFGVN QIGCTYSIRFRGVSVTISGGSRTLQHLCEMAIRSKQEL LQLTPVEVESNVSRGCPEGVETFEEESE |

### 1. Construction of collaaen gene expression cassette

A synthetic gene encoding Collagen alpha-1 (I) chain (Col1, SEQ ID NO: 8) fused to a barley amylase signal peptide (Barley AMY 1.2 Signal Peptide, SEQ ID NO: 5) was cloned in an expression cassette consisting of the CaMV 35S promoter (SEQ ID NO: 1), the Arabidopsis ADH-derived 5'-UTR (SEQ ID NO: 3) and a HSP terminator (SEQ ID NO: 4) (Fig. 1a). An expression cassette of Col1 was constructed in which a mutation was introduced into Col1 to make modifications such as modification of an N-terminus or C-terminus pro region cleavage site (introduction of cleavage sites by HRV3C, Furin or Enterokinase), removal of the N-terminus pro region, and replacement of the N-terminus pro region with GFP (SEQ ID NO: 13).

Specifically, the N-terminus pro region cleavage site was modified by replacing the C-terminus region of the N-terminus pro region with a recognition sequence for HRV3C, Furin or Enterokinase. Here, Furin and Enterokinase cleave the C-terminus of the recognition sequence, and the procollagen after the cleavage treatment is collagen having a natural N-terminus telopeptide sequence. On the other hand, HRV3C cleaves the inside of the recognition sequence, and the procollagen after the cleavage treatment is collagen having an N-terminus telopeptide sequence to which GlyPro, a C-terminus sequence of the recognition sequence for HRV3C, is added. Inside the plant cell, the cleavage occurs at a location closer by two amino acid residues (GlnLeu) to the C-terminus than the natural N-terminus cleavage site. As a modification for reproducing the cleavage inside the plant cell, a recognition sequence for Enterokinase was introduced into a region extending across the N-terminus pro region and the N-terminus telopeptide region to also construct an expression cassette of Col1, which produces collagen having an N-terminus telopeptide sequence in which two amino acid residues (GlnLeu) at the N-terminus are deleted.

Since modification of the C-terminus pro region removes recognition sequences for unknown proteases that cleave the C-terminus pro region inside the plant cell, a plurality of expression cassettes were constructed in which amino acids were substituted for the recognition sequence for Enterokinase at different locations from the C-terminus telopeptide region to the C-terminus pro region.

A synthetic gene encoding Collagen alpha-2 (I) chain (Col2, SEQ ID NO: 11) fused to a barley amylase signal peptide (SEQ ID NO: 5) was cloned in an expression cassette consisting of the CaMV 35S promoter (SEQ ID NO: 1), the Arabidopsis ADH-derived 5'-UTR (SEQ ID NO: 3) and a HSP terminator (SEQ ID NO: 4) (Fig. 1b). An expression cassette of Col2 was constructed in which a mutation was introduced into Col2 to make modifications such as modification of an N-terminus or C-terminus pro region cleavage site, removal of the N-terminus pro region, fusion of a green fluorescent protein to the N-terminus, and addition of a HN tag (SEQ ID NO: 16), a GST tag (SEQ ID NO: 14) and a MBP tag (SEQ ID NO: 15). The details of the modifications were the same as the modifications of the expression cassette of Col1.

### 3. Transformation of Agrobacterium

The Agrobacterium GV3101 strain was transformed by a freezing and thawing method using a plasmid derived from the pRI 201-AN plant transformation vector prepared as described in the above section 2. The Agrobacterium transformed with each plasmid was used for infiltration under conditions in which one strain was used alone or two strains were mixed.

The Nicotiana benthamiana plant was grown from seeds in plug trays filled with commercially available coconut shell substrates. The plants were grown in a greenhouse under a light cycle of lightness for 16 h/darkness for 8 h and a temperature schedule of 25°C all day. The agroinfiltration was performed on the plant 4 weeks after the seeding. Immediately before the agroinfiltration, the buds were picked from the plant to remove the apical buds.

### 2. Construction of modified enzyme expression vector

A synthetic gene encoding human P4Hα (SEQ ID NO: 17), human P4Hβ (SEQ ID NO: 19), nematode P4Hα (SEQ ID NO: 18) and mouse P4Hβ (SEQ ID NO: 20) fused to a vacuolar signal (Thiol protease aleurain (Barley) vacuolar signal, SEQ ID NO: 6) or a barley amylase signal peptide (SEQ ID NO: 5) was cloned in an expression cassette consisting of the CaMV 35S promoter (SEQ ID NO: 1) and the Arabidopsis ADH-derived 5'-UTR (SEQ ID NO: 3), the TMV omega sequence (SEQ ID NO: 2), a HSP-derived terminator (SEQ ID NO: 4) (Fig. 1c). The KDEL sequence was removed from the 3'-terminus of the P4Hβ gene except for the case of accumulation in the endoplasmic reticulum.

A synthetic gene encoding LH3 (SEQ ID NO: 21) fused to the vacuolar signal (SEQ ID NO: 6) or the barley amylase signal peptide (SEQ ID NO: 5) was cloned in an expression cassette consisting of the CaMV 35S promoter (SEQ ID NO: 1), the Arabidopsis ADH-derived 5'-UTR (SEQ ID NO: 3) and a HSP terminator (SEQ ID NO: 4) (Fig. 1d).

A synthetic gene encoding BMP1 (SEQ ID NO: 22) fused to a HN tag (SEQ ID NO: 16) fused to the vacuolar signal (SEQ ID NO: 6) or the barley amylase signal peptide (SEQ ID NO: 5) was cloned in an expression cassette consisting of the CaMV 35S promoter (SEQ ID NO: 1), the Arabidopsis ADH-derived 5'-UTR (SEQ ID NO: 3) and a HSP terminator (SEQ ID NO: 4) (Fig. 1f). A KDEL sequence was added to the 3' end of the BMP1 gene only in the case of accumulation in the endoplasmic reticulum.

A synthetic gene (SEQ ID NO: 23) encoding the Tomato bushy stunt virus-derived RNA silencing suppressor p19 (UniProtKB-P50628) was cloned in an expression cassette consisting of the CaMV 35S promoter (SEQ ID NO: 1), the Arabidopsis ADH-derived 5'-UTR (SEQ ID NO: 3) and HSP-derived terminator (SEQ ID NO: 4) (Fig. 1d).

The constructed expression cassettes of genes were cloned in the following combinations in the multi-cloning site of the pRI 201-AN plant transformation vector.
(1)Col1+p19
(2)Col2+p19
(3)Col1+Col2+P4Ha+P4Hβ+LH3+p19
(4)Col1+Col2+p19
(5)P4Hα+P4Hβ+LH3+p19
(6)Col1+P4Hα+P4Hβ+p19
(7)Col1+LH3+p19
(8)Col1+BMP1+p19

### 4. Acquisition of collagen from Agrobacterium

Agrobacterium transformed with each plasmid was shake-cultured overnight at 28°C in Select Alternative Protein Source (APS) medium containing 50 µg/ml kanamycin, and grown until the OD₆₀₀ reached 4.5 to 5.5. The Agrobacterium culture solution was centrifuged and the pellets were resuspended in an infiltration buffer (10mM MgCl₂ and 10mM MES (pH5.6)). The concentration of the bacteria liquid was adjusted to an OD₆₀₀ of 0.4 (0.8 in the case of simultaneous inoculation of two strains). The plant after infiltration was incubated for 6 days, and only Agrobacterium-infiltrated leaves were harvested. The harvested leaves were frozen at -80°C and crushed into small pieces.

The total soluble protein was extracted by adding, to about 0.1 g of the frozen and ground leaf, three times its volume of 50 mM Tris (pH 7.4), and 0.15 M NaCl, and grinding the mixture, followed by centrifugation at 20,000 g for 5 minutes at 4°C. As another extraction condition, three times the volume of the leaf of 0.5 M acetic acid, and 0.2 M NaCl were used.

### 5. Analysis of acquired collagen

The collagen extracted under the following conditions was treated, and whether cleavage occurred or not was confirmed by SDS-PAGE.
Treatment of collagen extract with protease: reaction at 4°C for 16 hours
HRV3C: 20 µL of extract + 1 µL of HRV3C (Millipore#71493)
Furin: 20 µL of the extract + 4 µL of 6mM CaCl₂ + 2 µL of Furin (R & D #1503-SE-010) diluted 10-fold
Enterokinase: 20 µL of extract +10 µL of 6mM CaCl₂ + 2 µL of Enterokinase (NEB#P8070S) diluted 10-fold

### 6. Mass spectrometry

Mass spectrometry samples were separated by reducing total soluble proteins by DTT, and then performing SDS-PAGE electrophoresis using MOPS buffer. After the electrophoresis, the separated proteins were detected by CBB staining, and a band presumed to originate from collagen was observed near about 130 kDa in a total soluble protein that expressed collagen. A gel of the corresponding portion was cut out, and mass spectrometry was performed on the protein contained. For the mass spectrometry, analysis was entrusted to Nihon Proteomics, Inc. The gel fragment was decolored, then digested with trypsin, followed by measurement of the mass by nanoLC-MS/MS. Thereafter, the resulting data was subjected to MascotSearch to identify proteins (https://www.jproteomics.com/).

### 7. Confirmation of expression of collagen in nicotiana leaves using Western-blot method

Total soluble proteins extracted from nicotiana leaves (Nicotiana benthamiana) were separated by performing SDS-PAGE electrophoresis using a Tris-glycine buffer solution. After the electrophoresis, the separated proteins were transferred to a PVDF film, and a Western-blot method was carried out with antibodies against collagen α1 and tag sequences to confirm the expression of collagen α1 and collagen α2.

### (Results)

### Example 1: Transient expression of secretory type I collagen α1 in plant expression system

By the above-described method, the Col1 expression cassette was transiently expressed in the Nicotiana benthamiana plant expression system, and whether cleavage occurred or not was confirmed by SDS-PAGE (Fig. 2). Samples for respective lanes are shown below.

| Lane | Sample |
|---|---|
| 1 | Marker |
| 2 | Negative control (negacon) extract |
| 3 | Procollagen α1 extract |
| 4 | N-terminus pro region-removed collagen α1 extract |
| 5 | N-terminus cleavage site-modified (HRV3C) procollagen α1 extract |
| 6 | N-terminus cleavage site-modified (HRV3C) procollagen α1 extract + HRV3C treatment |
| 7 | N-terminus cleavage site-modified (Furin) procollagen α1 extract |
| 8 | N-terminus cleavage site-modified (Furin) procollagen α1 extract + Furin treatment |
| 9 | N-terminus cleavage site-modified (Enterokinase) procollagen α1 extract |
| 10 | N-terminus cleavage site-modified (Enterokinase) procollagen α1 extract + Enterokinase treatment |
| 11 | N-terminus cleavage site-modified (cleavage location inside plant)(Enterokinase) procollagen α1 extract |
| 12 | N-terminus cleavage site-modified (cleavage location inside plant)(Enterokinase) procollagen α1 extract + Enterokinase treatment |
| 13 | N-terminus GFP bond cleavage site-modified (HRV3C) procollagen α1 extract |
| 14 | N-terminus GFP bond cleavage site-modified (HRV3C) procollagen α1 extract + HRV3C treatment |
| 15 | N-terminus GFP bond cleavage site-modified (Enterokinase) procollagen α1 extract |
| 16 | N-terminus GFP bond cleavage site-modified (Enterokinase) procollagen α1 extract + HRV3C treatment |
| 17 | C-terminus cleavage site-modified (Enterokinase) procollagen α1 extract |
| 18 | C-terminus cleavage site-modified (Enterokinase) procollagen α1 extract + HRV3C treatment |

The results showed that a full-length collagen protein having an N-terminus pro region and a C-terminus pro region was accumulated inside the plant body (Fig. 2, lane 3). The results were unexpected as they are in contrast to the experimental results of studies by F Ruggiero et al., 2000, which show that the N-terminus pro region and the C-terminus pro region of secretory type I collagen α1 are cleaved in a plant stable expression system. In addition, it was shown that it was possible to cleave the N-terminus and C-terminus pro regions of type I collagen α1N by treating type I collagen α1 having a cleavage site modification with an enzyme corresponding to the cleavage site modification in the extract (Fig. 2, lanes 4 to 18).

### Example 2: Acquisition of GFP-fused collagen α1

Fluorescence was confirmed by irradiating an extract of GFP-fused collagen α1 extract with excitation light. Of the N-terminus and C-terminus pro regions, the C-terminus pro region is considered necessary for collagen to form a triple-helical chain, whereas the N-terminus pro region is not absolutely necessary. In fact, the expression has been confirmed even in a sequence where the N-terminus pro region is removed. The present experiment showed that it was possible to produce collagen with a new function by fusing a heterogeneous peptide sequence such as GFP instead of the N-terminus pro region (Fig. 3).

### Example 3: Confirmation of co-expression of collagen α1 and collagen α2

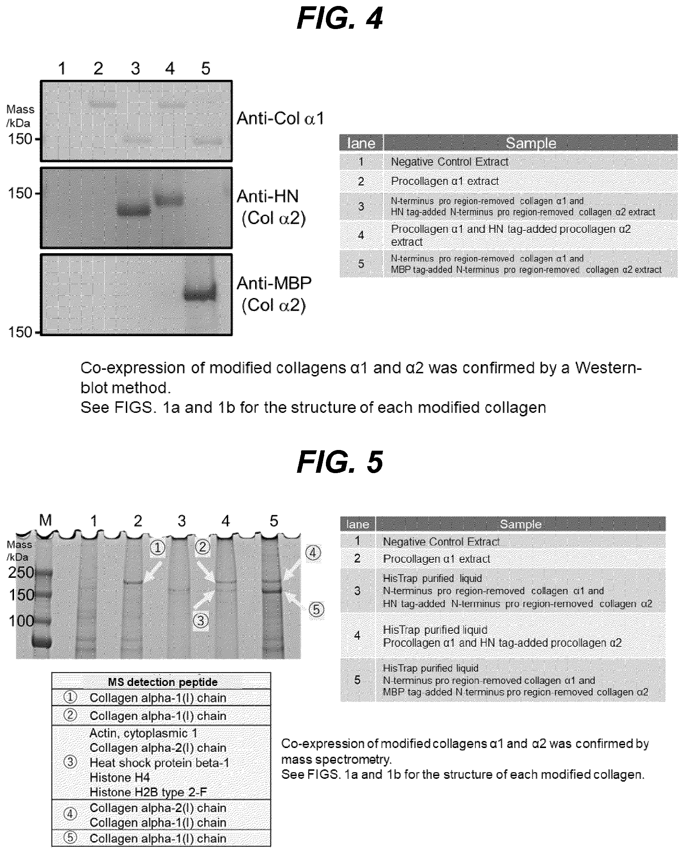

The expression cassette of Col1 and the expression cassette of Col2 were transiently co-expressed in a plant expression system by the above-described method, and it was confirmed by a Western-blot method that α1 and α2 collagen proteins were simultaneously accumulated inside the cell body (Fig. 4). Samples for respective lanes are shown below.

| Lane | Sample |
|---|---|
| 1 | Negative control (negacon) extract |
| 2 | Procollagen α1 extract |
| 3 | HisTrap purified liquid |
| | N-terminus pro region-removed collagen α1 and |
| | HN tag-added N-terminus pro region-removed collagen α2 |
| 4 | HisTrap purified liquid |
| | Procollagen α1 and |
| | HN tag-added procollagen α2 |
| 5 | HisTrap purified liquid |
| | N-terminus pro region-removed collagen α1 and |
| | MBP tag-added N-terminus pro region-removed collagen α2 |

It was also confirmed by mass spectrometry that α1 and α2 collagen proteins were expressed (Fig. 5). Samples for respective lanes are the same as in Fig. 4.

From the above examples, it was surprisingly found that it was possible to stably acquire a secretory collagen by transiently expressing a collagen protein in a plant expression system. In addition, it was confirmed that it was also possible to acquire high-functionality collagen by expressing collagen to which peptides having various functions are fused instead of the N-terminus pro region.
<Seq ID No:1 CaMV 35S Promoter DNA Seq; DNA; Cauliflower mosaic virus> 
TGAGACTTTTCAACAAAGGGTAATATCCGGAAACCTCCTCGGATTCCATTGCCCAGCTATCTGTCAC
TTTATTGTGAAGATAGTGGAAAAGGAAGGTGGCTCCTACAAATGCCATCATTGCGATAAAGGAAAGG
CCATCGTTGAAGATGCCTCTGCCGACAGTGGTCCCAAAGATGGACCCCCACCCACGAGGAGCATCGT
GGAAAAAGAAGACGTTCCAACCACGTCTTCAAAGCAAGTGGATTGATGTGATATCTCCACTGACGTA
AGGGATGACGCACAATCCCACTATCCTTCGCAAGACCCTTCCTCTATATAAGGAAGTTCATTTCATT
TGGAGAGAACA
<Seq ID No:2 TMV omega DNA Seq; DNA; Tobacco mosaic virus>
   ACAATTACCAACAACAACAAACAACAAACAACATTACAATTACTATTTACAATTAC
<Seq ID No:3 AtADH 5'-UTR DNASeq; DNA; Arabidopsis thaliana>
   TACATCACAATCACACAAAACTAACAAAAGATCAAAAGCAAGTTCTTCACTGTTGATA
<Seq ID No:4 HSP-terminator DNA Seq; DNA; Arabidopsis thaliana> 
TATGAAGATGAAGATGAAATATTTGGTGTGTCAAATAAAAAGCTAGCTTGTGTGCTTAAGTTTGTGT
TTTTTTCTTGGCTTGTTGTGTTATGAATTTGTGGCTTTTTCTAATATTAAATGAATGTAAGATCTCA
TTATAATGAATAAACAAATGTTTCTATAATCCATTGTGAATGTTTTGTTGGATCTCTTCGCATATAA
CTACTGTATGTGCTATGGTATGGACTATGGAATATGATTAAAGATAAG
<Seq ID No:5 Barley AMY 1.2 Signal Peptide AA Seq; AA; Hordeum vulgare>
   MANKHLSLSLFLVLLGLSASLASG
<Seq ID No:6 Thiol protease aleurain (Barley) vacuolar signal AA Seq; AA; Hordeum vulgare>
   MAHARVLLLALAVLATAAVAVASSSSFADSNPTRPVTDRAASTL
<Seq ID No:7 Collagen alpha-1 N-terminal propeptide AA Seq; AA; Homo sapiens> 
QEEGQVEGQDEDIPPITCVQNGLRYHDRDVWKPEPCRICVCDNGKVLCDDVICDETKNCPGAEVPEG
ECCPVCPDGSESPTDQETTGVEGPKGDTGPRGPRGPAGPPGRDGIPGQPGLPGPPGPPGPPGPPGLG
GNFAP
<Seq ID No:8 Collagen alpha-1 (T) chain AA Seq; AA; Homo sapiens> 
QEEGQVEGQDEDIPPITCVQNGLRYHDRDVWKPEPCRICVCDNGKVLCDDVICDETKNCPGAEVPEG
ECCPVCPDGSESPTDQETTGVEGPKGDTGPRGPRGPAGPPGRDGIPGQPGLPGPPGPPGPPGPPGLG
GNFAPQLSYGYDEKSTGGISVPGPMGPSGPRGLPGPPGAPGPQGFQGPPGEPGEPGASGPMGPRGPP
GPPGKNGDDGEAGKPGRPGERGPPGPQGARGLPGTAGLPGMKGHRGFSGLDGAKGDAGPAGPKGEPG
SPGENGAPGQMGPRGLPGERGRPGAPGPAGARGNDGATGAAGPPGPTGPAGPPGFPGAVGAKGEAGP
QGPRGSEGPQGVRGEPGPPGPAGAAGPAGNPGADGQPGAKGANGAPGIAGAPGFPGARGPSGPQGPG
GPPGPKGNSGEPGAPGSKGDTGAKGEPGPVGVQGPPGPAGEEGKRGARGEPGPTGLPGPPGERGGPG
SRGFPGADGVAGPKGPAGERGSPGPAGPKGSPGEAGRPGEAGLPGAKGLTGSPGSPGPDGKTGPPGP
AGQDGRPGPPGPPGARGQAGVMGFPGPKGAAGEPGKAGERGVPGPPGAVGPAGKDGEAGAQGPPGPA
GPAGERGEQGPAGSPGFQGLPGPAGPPGEAGKPGEQGVPGDLGAPGPSGARGERGFPGERGVQGPPG
PAGPRGANGAPGNDGAKGDAGAPGAPGSQGAPGLQGMPGERGAAGLPGPKGDRGDAGPKGADGSPGK
DGVRGLTGPIGPPGPAGAPGDKGESGPSGPAGPTGARGAPGDRGEPGPPGPAGFAGPPGADGQPGAK
GEPGDAGAKGDAGPPGPAGPAGPPGPIGNVGAPGAKGARGSAGPPGATGFPGAAGRVGPPGPSGNAG
PPGPPGPAGKEGGKGPRGETGPAGRPGEVGPPGPPGPAGEKGSPGADGPAGAPGTPGPQGIAGQRGV
VGLPGQRGERGFPGLPGPSGEPGKQGPSGASGERGPPGPMGPPGLAGPPGESGREGAPGAEGSPGRD
GSPGAKGDRGETGPAGPPGAPGAPGAPGPVGPAGKSGDRGETGPAGPTGPVGPVGARGPAGPQGPRG
DKGETGEQGDRGIKGHRGFSGLQGPPGPPGSPGEQGPSGASGPAGPRGPPGSAGAPGKDGLNGLPGP
IGPPGPRGRTGDAGPVGPPGPPGPPGPPGPPSAGFDFSFLPQPPQEKAHDGGRYYRADDANVVRDRD
LEVDTTLKSLSQQIENIRSPEGSRKNPARTCRDLKMCHSDWKSGEYWIDPNQGCNLDAIKVFCNMET
GETCVYPTQPSVAQKNWYISKNPKDKRHVWFGESMTDGFQFEYGGQGSDPADVAIQLTFLRLMSTEA
SQNITYHCKNSVAYMDQQTGNLKKALLLQGSNEIEIRAEGNSRFTYSVTVDGCTSHTGAWGKTVIEY
KTTKTSRLPIIDVAPLDVGAPDQEFGFDVGPVCFL
<Seq ID No:9 Collagen alpha-1 C-terminal propeptide AA Seq; AA; synthetic construct> 
DDANVVRDRDLEVDTTLKSLSQQIENIRSPEGSRKNPARTCRDLKMCHSDWKSGEYWIDPNQGCNLD
AIKVFCNMETGETCVYPTQPSVAQKNWYISKNPKDKRHVWFGESMTDGFQFEYGGQGSDPADVAIQL
TFLRLMSTEASQNITYHCKNSVAYMDQQTGNLKKALLLQGSNEIEIRAEGNSRFTYSVTVDGCTSHT
GAWGKTVIEYKTTKTSRLPIIDVAPLDVGAPDQEFGFDVGPVCFL
<Seq ID No:10 Collagen alpha-2 N-terminal propeptide AA Seq; AA; synthetic construct>
   QSLQEETVRKGPAGDRGPRGERGPPGPPGRDGEDGPTGPPGPPGPPGPPGLGGNFAA
<Seq ID No:11 Collagen alpha-2 (I) chain AA seq; AA; Homo sapiens> QSLQEETVRKGPAGDRGPRGERGPPGPPGRDGEDGPTGPPGPPGPPGPPGLGGNFAAQYDGKGVGLG 
PGPMGLMGPRGPPGAAGAPGPQGFQGPAGEPGEPGQTGPAGARGPAGPPGKAGEDGHPGKPGRPGER
GVVGPQGARGFPGTPGLPGFKGIRGHNGLDGLKGQPGAPGVKGEPGAPGENGTPGQTGARGLPGERG
RVGAPGPAGARGSDGSVGPVGPAGPIGSAGPPGFPGAPGPKGEIGAVGNAGPAGPAGPRGEVGLPGL
SGPVGPPGNPGANGLTGAKGAAGLPGVAGAPGLPGPRGIPGPVGAAGATGARGLVGEPGPAGSKGES
GNKGEPGSAGPQGPPGPSGEEGKRGPNGEAGSAGPPGPPGLRGSPGSRGLPGADGRAGVMGPPGSRG
ASGPAGVRGPNGDAGRPGEPGLMGPRGLPGSPGNIGPAGKEGPVGLPGIDGRPGPIGPAGARGEPGN
IGFPGPKGPTGDPGKNGDKGHAGLAGARGAPGPDGNNGAQGPPGPQGVQGGKGEQGPPGPPGFQGLP
GPSGPAGEVGKPGERGLHGEFGLPGPAGPRGERGPPGESGAAGPTGPIGSRGPSGPPGPDGNKGEPG
VVGAVGTAGPSGPSGLPGERGAAGIPGGKGEKGEPGLRGEIGNPGRDGARGAPGAVGAPGPAGATGD
RGEAGAAGPAGPAGPRGSPGERGEVGPAGPNGFAGPAGAAGQPGAKGERGAKGPKGENGVVGPTGPV
GAAGPAGPNGPPGPAGSRGDGGPPGMTGFPGAAGRTGPPGPSGISGPPGPPGPAGKEGLRGPRGDQG
PVGRTGEVGAVGPPGFAGEKGPSGEAGTAGPPGTPGPQGLLGAPGILGLPGSRGERGLPGVAGAVGE
PGPLGIAGPPGARGPPGAVGSPGVNGAPGEAGRDGNPGNDGPPGRDGQPGHKGERGYPGNIGPVGAA
GAPGPHGPVGPAGKHGNRGETGPSGPVGPAGAVGPRGPSGPQGIRGDKGEPGEKGPRGLPGLKGHNG
LQGLPGIAGHHGDQGAPGSVGPAGPRGPAGPSGPAGKDGRTGHPGTVGPAGIRGPQGHQGPAGPPGP
PGPPGPPGVSGGGYDFGYDGDFYRADQPRSAPSLRPKDYEVDATLKSLNNQIETLLTPEGSRKNPAR
TCRDLRLSHPEWSSGYYWIDPNQGCTMDAIKVYCDFSTGETCIRAQPENIPAKNWYRSSKDKKHVWL
GETINAGSQFEYNVEGVTSKEMATQLAFMRLLANYASQNITYHCKNSIAYMDEETGNLKKAVILQGS
NDVELVAEGNSRFTYTVLVDGCSKKTNEWGKTIIEYKTNKPSRLPFLDIAPLDIGGADQEFFVDIGP
VCFK
<Seq ID No:12 Collagen alpha-2 C-terminal propeptide AA Seq;AA; synthetic construct> 
DQPRSAPSLRPKDYEVDATLKSLNNQIETLLTPEGSRKNPARTCRDLRLSHPEWSSGYYWIDPNQGC
TMDAIKVYCDFSTGETCIRAQPENIPAKNWYRSSKDKKHVWLGETINAGSQFEYNVEGVTSKEMATQ
LAFMRLLANYASQNITYHCKNSIAYMDEETGNLKKAVILQGSNDVELVAEGNSRFTYTVLVDGCSKK
TNEWGKTIIEYKTNKPSRLPFLDIAPLDIGGADQEFFVDIGPVCFK
<Seq ID No:13 GFP AA Seq; AA; synthetic construct> 
SKGEELFTGVVPILVELDGDVNGHKFSVSGEGEGDATYGKLTLKFICTTGKLPVPWPTLVTTFSYGV
QCFSRYPDHMKQHDFFKSAMPEGYVQERTIFFKDDGNYKTRAEVKFEGDTLVNRIELKGIDFKEDGN
ILGHKLEYNYNSHNVYIMADKQKNGIKVNFKIRHNIEDGSVQLADHYQQNTPIGDGPVLLPDNHYLS
TQSALSKDPNEKRDHMVLLEFVTAAGITHGMDELYK
<Seq ID No:14 GST AA Seq;AA; synthetic construct> SPILGYWKIKGLVQPTRLLLEYLEEKYEEHLYERDEGDKWRNKKFELGLEFPNLPYYIDGDVKLTQS 
MAIIRYIADKHNMLGGCPKERAEISMLEGAVLDIRYGVSRIAYSKDFETLKVDFLSKLPEMLKMFED
RLCHKTYLNGDHVTHPDFMLYDALDVVLYMDPMCLDAFPKLVCFKKRIEAIPQIDKYLKSSKYIAWP
LQGWQATFGGGDHPPK
<Seq ID No:15 MBP AA Seq; AA; Escherichia coli> 
KIEEGKLVIWINGDKGYNGLAEVGKKFEKDTGIKVTVEHPDKLEEKFPQVAATGDGPDIIFWAHDRF
GGYAQSGLLAEITPDKAFQDKLYPFTWDAVRYNGKLIAYPIAVEALSLIYNKDLLPNPPKTWEEIPA
LDKELKAKGKSALMFNLQEPYFTWPLIAADGGYAFKYENGKYDIKDVGVDNAGAKAGLTFLVDLIKN
KHMNADTDYSIAEAAFNKGETAMTINGPWAWSNIDTSKVNYGVTVLPTFKGQPSKPFVGVLSAGINA
ASPNKELAKEFLENYLLTDEGLEAVNKDKPLGAVALKSYEEELVKDPRIAATMENAQKGEIMPNIPQ
MSAFWYAVRTAVINAASGRQTVDEALKDAQTNSSSNNNNNNNNNNLG
<Seq ID No:16 HN Tag AA Seq; AA; synthetic construct>
   HNHNHNHNHNHN
<Seq ID No:17 Human P4H alpha subunit AA Seq; AA; Homo sapiens> 
HPGFFTSIGQMTDLIHTEKDLVTSLKDYIKAEEDKLEQIKKWAEKLDRLTSTATKDPEGFVGHPVNA
FKLMKRLNTEWSELENLVLKDMSDGFISNLTIQRQYFPNDEDQVGAAKALLRLQDTYNLDTDTISKG
NLPGVKHKSFLTAEDCFELGKVAYTEADYYHTELWMEQALRQLDEGEISTIDKVSVLDYLSYAVYQQ
GDLDKALLLTKKLLELDPEHQRANGNLKYFEYIMAKEKDVNKSASDDQSDQKTTPKKKGVAVDYLPE
RQKYEMLCRGEGIKMTPRRQKKLFCRYHDGNRNPKFILAPAKQEDEWDKPRIIRFHDIISDAEIEIV
KDLAKPRLRRATISNPITGDLETVHYRISKSAWLSGYENPVVSRINMRIQDLTGLDVSTAEELQVAN
YGVGGQYEPHFDFARKDEPDAFKELGTGNRIATWLFYMSDVSAGGATVFPEVGASVWPKKGTAVFWY
NLFASGEGDYSTRHAACPVLVGNKWVSNKWLHERGQEFRRPCTLSELE
<Seq ID No:18 C. elegans P4H alpha subunit AA Seq; AA; Caenorhabditis elegans> 
DLFTSIADMQNLLETERNIPKILDKYIHDEEERLVQLKKLSEEYSKKNEISIENGLKDITNPINAFL
LIKRKIFDWKEIESKMNANKAGNVVSSITDDSYGVRYPTADDLSGAAIGLLRLQDTYRLDTKDLADG
KIYADQGNYTFSAKDCFEIARAAYNEHDFYHTVMWMEEAQRRLGDEVEPTVEVEDILEYLAFALYKQ
NNLKHALKLTEELYKMNPTHPRAKGNVKWYEDLLEQEGVRRSDMRKNLPPIQNRRPDSVLGNTERTM
YEALCRNEVPVSQKDISRLYCYYKRDRPFLVYAPIKVEIKRFNPLAVLFKDVISDDEVAAIQELAKP
KLARATVHDSVTGKLVTATYRISKSAWLKEWEGDVVETVNKRIGYMTNLEMETAEELQIANYGIGGH
YDPHFDHAKKEESKSFESLGTGNRIATVLFYMSQPSHGGGTVFTEAKSTILPTKNDALFWYNLYKQG
DGNPDTRHAACPVLVGIKWVSNKWIHEKGNEFRRPCGLKSSDYERFVGDLGYGPEPRNAPNVSPNLA
KDVWETL
<Seq ID No:19 Human P4H beta subunit AA Seq; AA; Homo sapiens> 
DAPEEEDHVLVLRKSNFAEALAAHKYLLVEFYAPWCGHCKALAPEYAKAAGKLKAEGSEIRLAKVDA
TEESDLAQQYGVRGYPTIKFFRNGDTASPKEYTAGREADDIVNWLKKRTGPAATTLPDGAAAESLVE
SSEVAVIGFFKDVESDSAKQFLQAAEAIDDIPFGITSNSDVFSKYQLDKDGVVLFKKFDEGRNNFEG
EVTKENLLDFIKHNQLPLVIEFTEQTAPKIFGGEIKTHILLFLPKSVSDYDGKLSNFKTAAESFKGK
ILFIFIDSDHTDNQRILEFFGLKKEECPAVRLITLEEEMTKYKPESEELTAERITEFCHRFLEGKIK
PHLMSQELPEDWDKQPVKVLVGKNFEDVAFDEKKNVFVEFYAPWCGHCKQLAPIWDKLGETYKDHEN
IVIAKMDSTANEVEAVKVHSFPTLKFFPASADRTVIDYNGERTLDGFKKFLESGGQDGAGDDDDLED
LEEAEEPDMEEDDDQKAV
<Seq ID No:20 Mouse P4H beta subunit AA Seq; AA; Mus musculus> 
DALEEEDNVLVLKKSNFEEALAAHKYLLVEFYAPWCGHCKALAPEYAKAAAKLKAEGSEIRLAKVDA
TEESDLAQQYGVRGYPTIKFFKNGDTASPKEYTAGREADDIVNWLKKRTGPAATTLSDTAAAESLVD
SSEVTVIGFFKDVESDSAKQFLLAAEAIDDIPFGITSNSGVFSKYQLDKDGVVLFKKFDEGRNNFEG
EITKEKLLDFIKHNQLPLVIEFTEQTAPKIFGGEIKTHILLFLPKSVSDYDGKLSSFKRAAEGFKGK
ILFIFIDSDHTDNQRILEFFGLKKEECPAVRLITLEEEMTKYKPESDELTAEKITEFCHRFLEGKIK
PHLMSQEVPEDWDKQPVKVLVGANFEEVAFDEKKNVFVEFYAPWCGHCKQLAPIWDKLGETYKDHEN
IIIAKMDSTANEVEAVKVHSFPTLKFFPASADRTVIDYNGERTLDGFKKFLESGGQDGAGDDEDLDL
EEALEPDMEEDDDQKAVKDEL
<Seq ID No:21 LH3 AA Seq; AA; Homo sapiens> 
SDRPRGRDPVNPEKLLVITVATAETEGYLRFLRSAEFFNYTVRTLGLGEEWRGGDVARTVGGGQKVR
WLKKEMEKYADREDMIIMFVDSYDVILAGSPTELLKKFVQSGSRLLFSAESFCWPEWGLAEQYPEVG
TGKRFLNSGGFIGFATTIHQIVRQWKYKDDDDDQLFYTRLYLDPGLREKLSLNLDHKSRIFQNLNGA
LDEVVLKFDRNRVRIRNVAYDTLPIVVHGNGPTKLQLNYLGNYVPNGWTPEGGCGFCNQDRRTLPGG
QPPPRVFLAVFVEQPTPFLPRFLQRLLLLDYPPDRVTLFLHNNEVFHEPHIADSWPQLQDHFSAVKL
VGPEEALSPGEARDMAMDLCRQDPECEFYFSLDADAVLTNLQTLRILIEENRKVIAPMLSRHGKLWS
NFWGALSPDEYYARSEDYVELVQRKRVGVWNVPYISQAYVIRGDTLRMELPQRDVFSGSDTDPDMAF
CKSFRDKGIFLHLSNQHEFGRLLATSRYDTEHLHPDLWQIFDNPVDWKEQYIHENYSRALEGEGIVE
QPCPDVYWFPLLSEQMCDELVAEMEHYGQWSGGRHEDSRLAGGYENVPTVDIHMKQVGYEDQWLQLL
RTYVGPMTESLFPGYHTKARAVMNFVVRYRPDEQPSLRPHHDSSTFTLNVALNHKGLDYEGGGCRFL
RYDCVISSPRKGWALLHPGRLTHYHEGLPTTWGTRYIMVSFVDP
<Seq ID No:22 BMP1 AA seq; AA; Homo sapiens> AATSRPERVWPDGVIPFVIGGNFTGSQRAVFRQAMRHWEKHTCVTFLERTDEDSYIVFTYRPCGCCS 
YVGRRGGGPQAISIGKNCDKFGIVVHELGHVVGFWHEHTRPDRDRHVSIVRENIQPGQEYNFLKMEP
QEVESLGETYDFDSIMHYARNTFSRGIFLDTIVPKYEVNGVKPPIGQRTRLSKGDIAQARKLYKCPA
CGETLQDSTGNFSSPEYPNGYSAHMHCVWRISVTPGEKIILNFTSLDLYRSRLCWYDYVEVRDGFWR
KAPLRGRFCGSKLPEPIVSTDSRLWVEFRSSSNWVGKGFFAVYEAICGGDVKKDYGHIQSPNYPDDY
RPSKVCIWRIQVSEGFHVGLTFQSFEIERHDSCAYDYLEVRDGHSESSTLIGRYCGYEKPDDIKSTS
SRLWLKFVSDGSINKAGFAVNFFKEVDECSRPNRGGCEQRCLNTLGSYKCSCDPGYELAPDKRRCEA
ACGGFLTKLNGSITSPGWPKEYPPNKNCIWQLVAPTQYRISLQFDFFETEGNDVCKYDFVEVRSGLT
ADSKLHGKFCGSEKPEVITSQYNNMRVEFKSDNTVSKKGFKAHFFSEKRPALQPPRGRPHQLKFRVQ
KRNRTPQ
<Scq ID No:23 RNA silencing suppressor p19 AA Seq; AA; Tomato bushy stunt virus> 
MERAIQGNDAREQANSERWDGGSGSSTSPFQLPDESPSWTEWRLHNDETNSNQDNPLGFKESWGFGK
VVFKRYLRYERTETSLHRVLGSWTGDSVNYAASRFFGVNQIGCTYSIRFRGVSVTISGGSRTLQHLC
EMAIRSKQELLQLTPVEVESNVSRGCPEGVETFEEESE
<Seq ID No:24 HRV3C recognition sequence ; AA; synthetic construct>
   LEVLFQGP
<Seq ID Ko:25 Furin recognition sequence 1; AA; synthetic construct>
   RXRR
<Seq ID Ko:26 Furin recognition sequence 2; AA; synthetic construct>
   RXKR
<Seq ID No:27 Enterokinase recognition sequence; AA; synthetic construct>
   DDDDK

## Claims

1. A method for producing procollagen or collagen from a plant body or an isolated plant cell, comprising steps of:
A) providing a plant body or an isolated plant cell containing a nucleic acid construct encoding procollagen;
B) transiently expressing procollagen in the plant body or the isolated plant cell; and
C) separating procollagen or collagen secreted to outside of the cell by step B.

2. A method for producing procollagen or collagen from a plant body or an isolated plant cell, comprising a step of cleaving an N-terminus or a C-terminus of procollagen secreted to outside of the cell after step B.

3. The method according to claim 1 or 2, wherein the nucleic acid construct contains a nucleic acid sequence encoding procollagen having an amino acid sequence identity of 80% or more to natural procollagen.

4. The method according to claim 1 or 2, wherein the nucleic acid construct contains a nucleic acid sequence encoding a full length of procollagen and has a mutation which prevents an N-terminus of the procollagen from being cleaved inside the plant cell.

5. Procollagen or collagen produced by the method according to claim 1 or 2.

6. A nucleic acid construct which expresses procollagen or collagen, and is used in the method according to claim 1.

7. An expression vector comprising the nucleic acid construct according to claim 6.

8. A plant body or an isolated plant cell comprising the nucleic acid construct according to claim 6 or the expression vector according to claim 7.

9. Procollagen or collagen encoded by the nucleic acid construct according to claim 6, the procollagen or collagen having a non-natural amino acid sequence.

10. The procollagen or collagen according to claim 9, wherein a heterogeneous peptide is fused to an N-terminus pro region.

11. A composition that is a pharmaceutical composition, a cosmetic composition or a food composition, the composition comprising procollagen or collagen produced by the method according to claim 1 or 2, or the procollagen or collagen according to claim 9 or 10.
